# EUROPEAN PATENT SPECIFICATION

(11) **EP 2 340 249 B1**
(45) Date of publication and mention of the grant of the patent: **11.09.2013**
(21) Application number: 09756849.7
(22) Date of filing: 02.11.2009
(51) Int. Cl.: C07D 413/14, A61K 31/444, A61P 35/00, A61K 31/4439, A61K 31/496

(54) **POLY-HETEROARYL DERIVATIVES FOR THE TREATMENT OF CANCER**
POLYHETEROARYLDERIVATE ZUR BEHANDLUNG VON KREBS
DÉRIVÉS DE POLY-HÉTÉROARYLE POUR LE TRAITEMENT DU CANCER

(30) Priority: 31.10.2008 EP 08168104
(43) Date of publication of application: 06.07.2011
(73) Proprietor: Institut Curie, 75248 Paris Cedex 05 (FR); Centre National de la Recherche Scientifique (CNRS), 75794 Paris Cedex 16 (FR); Institut National De La Sante Et De La Recherche Medicale, 75654 Paris Cedex 05 (FR); Museum National D'histoire Naturelle (MNHN), 75231 Paris cedex 05 (FR)
(72) Inventor: NGUYEN, Chi-Hung, F-92160 Antony (FR); ROUCHON DAGOIS, Myriam, F-91120 Palaiseau (FR); GUEDIN-BEAUREPAIRE, Aurore, F-91240 Saint-Michel-sur-Orge (FR); MONCHAUD, David, F-21000 Dijon (FR); TEULADE-FICHOU, Marie-Paule, F-75014 Paris (FR); RIOU, Jean-François, F-75014 Paris (FR); MERGNY, Jean-Louis, F-94800 Villejuif (FR); GRIERSON, David, Vancouver BC V6T 2L1 (CA)
(74) Representative: Gallois, Valérie
(86) International application number: PCT/IB2009/054856
(87) International publication number: WO 2010/049915

(56) References cited:
- EP-A- 1 746 094
- WO-A-2007/119461
- WO-A-2008/012440

## Description

The present invention relates to poly-heteroaryl derivatives that specifically bind and stabilise quadruplex DNA. The invention also relates to the pharmaceutically acceptable salts of such compounds, and processes for the preparation of pharmaceutical compositions containing them. "Polyheteroaryl"as used in the description and the claims means a sequence/chain consisting of aromatic heterocyclic moieties, optionally including phenylene moiety(ies) and/or having end terminal phenyl groups.

G-quadruplex DNA is currently considered as a structural element able to regulate the function of G-rich sequences. In particular, many lines of evidence suggest that formation of this peculiar DNA structure at telomeres or in specific gene promoters in a variety of human oncogenes, including c-Myc, Bcl-2, VEGF, Hif-1a, Ret, c-Kit, PDGF-A, KRAS and c-Myb (1) may inhibit cancer cell proliferation.

Telomeric DNA of human cells comprises tandem repeats of sequence 5'-TTAGGG-3', which terminates on their 3' side in a single-stranded overhang that was demonstrated to fold into G-quadruplex *in vitro.* The formation of such quadruplex-structure *in vivo* is hypothesized to lead to the displacement of protective proteins normally associated with telomeres (shelterin complex), therefore disrupting telomere structure leading to genomic instability.

For oncogenes promoters, stabilisation of quadruplex is expected to disrupt the local environment of proteins factors that regulates gene expression and has been well documented for c-myc (1). Hence chemical intervention able to induce or stabilize quadruplex-DNA formation has been thoroughly investigated *via* small molecules quadruplex binders, with the goal to control DNA-related functions (telomere elongation, oncogene transcription) in particular in cancer cells.

Compounds that interact with G-quadruplex-DNA are currently numerous in the literature, as illustrated by the number of recent reviews dedicated to them (2- 9) However only few compounds present a high degree of selectivity for quadruplex- *vs* duplex-DNA; additionally, only a few of these compounds have been thoroughly investigated as anti-cancer agents. The leading compound of this family is telomestatin, a naturally-occurring macrocycle (isolated from *Streptomyces anulatus* 3533-SV4) comprised of five oxazole rings, two methyl oxazole rings and a thiazoline ring. Telomestatin represents a paradigm in terms of quadruplex-selective interactions, and shows exceptional telomerase-inhibiting properties *in vitro* (evaluated through either TRAP or Direct assay, with IC₅₀-TRAP = 0.6nM and IC₅₀-Direct Assay = 58nM (10) .Additionally, telomestatin is also deeply investigated for its cellular effects and presents antiproliferative and apoptotic properties in various tumor cell lines (11 and 12). Telomestatin alters telomere integrity (13) and triggers a DNA-damage response at telomeres (14). Telomestatin induced POT1 uncapping *in vitro* (IC_{50-POTI} = 500 nM) and removes GFP-POT1 from telomeres in tumor cells (15). Given the harsh synthetic access of telomestatin (16), analogues have been reported (17, 18 and 19) that present interesting quadruplex-interacting properties.

WO2008/012440 discloses oligheteroaryl derivatives comprising at least one pyridazine or pyrrole moiety suitable as anti-cancer agents.

However, none of these compounds show the exceptional properties of telomestatin. Additionally, none of these compounds, including telomestatin itself, has been investigated for their fluorescence properties. Given the chemical instability, the poor water-solubility and in particular, the arduous synthesis of telomestatin, its large-scale therapeutic use is questionable.

In this context, the inventors have found that new non-macrocyclic polyheteroaryl derivatives exhibit an exquisite selectivity for quadruplex- over duplex-DNA and that the association of these derivatives with their DNA targets leads to i) deep modifications of their spectroscopic properties, ii) uncap human POT1 (protection of telomere 1) protein from the telomeric G-overhang, iii) inhibit cell proliferation of human tumour cell lines. The stability and solubility in physiological media of this new family of compounds are compatible with their use in therapeutic treatments. Moreover, the inventors have found an efficient and original process for obtaining these compounds.

An object of the present invention is then to provide, as new products, such non-macrocyclic polyheteroaryl derivatives.

It also relates to a method for their synthesis.

According to another object, the invention, taking advantage of the quadruplex-interacting properties of said compounds, further relates to the use of said derivatives as quadruplex-specific probes. In particular, in view of the high fluorescence quantum yield quantum of the disclosed coumpounds, such compounds could be used as fluorescent probes for the detection and/or purification of quadruplex DNA or related nucleic acid structure including but not limited to quadruplex RNA.

In still another object, the invention provides pharmaceutical compositions containing said derivatives as active principles and also relates to the use of said derivatives in the manufacture of drugs for a wide range of disorders, particularly for the treatment of patients suffering from, for example, cancer or infectious diseases such as malaria.

The polyheteroaryl derivatives of the invention are penta-, hexa-, hepta-, octa-, nona-and deca-heteroaryl derivatives (abbreviated hereafter as penta- to deca-heteroaryl) comprising
- a combination of heterocycle 1 (Het-1)ₐ and/or heterocycle 2 (Het-2)_{b} and/ or heterocycle 3 (Het-3)_{c} and/or heterocycle 4 (Het-4)_{d} of formulae I, II, III and IV respectively, 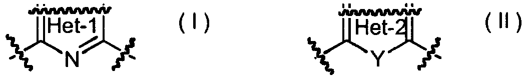 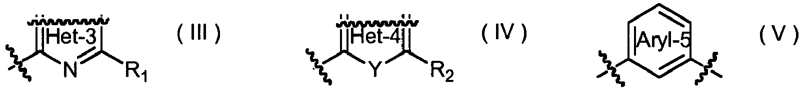 the N-oxides, the pharmaceutically acceptable addition salts,
- said combination comprising at least two different heterocyclic moieties, and optionally comprising an (Aryl-5)ₑ moiety of formula (V)
- a, b and e, being integers from 0 to 6, c and d being integers from 0 to 2, the sum a + b + c + d + e being ≤ 10
- Y is O or S;
- Het-1 is a class of nitrogen heterocyclic-diyl ring selected in the group comprising 2,6-pyridin-diyl or 2,4-pyrimidin-diyl or 3,5-pyrazin-diyl or 2,4-(1,3,5-triazin)diyl or 3,5-(1,2,4-triazin)diyl or 2,4-oxazolin-diyl or 2,4-thiazolin-diyl ,;
- Het-2 is a class of five-membered heterocyclic-diyl ring selected in the group comprising 2,5-oxazolin-diyl or 2,5-thiazolin-diyl, or 2,5-thiophen-diyl or 2,5-furan-diyl,;
- Het-3 and/ or Het-4 constitutes the endings of the said penta- hexa-, hepta-, octa-, nona- and deca-heteroaryl derivatives, wherein
- Het-3 is a class of nitrogen heterocyclic-yl ring selected from 2-pyridyl or 2-pyrimidyl or 4-pyrimidyl or 2-pyrazyl or 2-(1,3,5)triazyl or 3-(1,2,4)triazyl or 5-(1,2,4)triazyl or 4-oxazolyl or 4-thiazolyl ;
- Het-4 is a class of five-membered heterocyclic-yl selected from 2-oxazolyl or 5-oxazolyl or 2-thiazolyl or 5-thiazolyl or 2-thienyl or 2-furanyl 1;
- R₁ and R₂, are each independently selected from hydrogen; C₁₋₆alkyl ; C₁₋₄alkyloxy ; halo; hydroxy; hydroxymethyl; nitro ; amino ; mono-or di(C₁₋₄alkyl)amino ; C₁-₄alkylmethylamino ; mono-or di(C₁₋₄alkyl)amino(C₂₋₄alkyl)amino methyl; morpholin-4-yl C₂₋₄alkyloxy; piperazin-1-ylC₂₋₄alkyloxy; 4-C₁₋₄alkylpiperazin-1-yl, monocyclic or bicyclic selected from phenyl, benzyl, naphthyl.

According to an embodiment of the invention, a, b, c and d are integers from 0 to 3, e=0, and the sum a+b+c+d being ≤ 10.

According to an embodiment of the invention, one or several of the above moieties are substituted with one, two or three substituents, each independently selected from C₁₋₆alkyl ; C₁₋₄alkyloxy ; halo; hydroxy ; nitro; amino ; mono-or di(C₁₋₄alkyl)amino ; C₁-₄alkylmethylamino ; morpholin-4-ylC₂₋₄alkyloxy ; piperazin-1-ylC₂₋₄alkyloxy ; 4-C₁-₄alkylpiperazin-1-ylC₂₋₄alkyloxy ; monocyclic or bicyclic ring selected from phenyl, benzyl, naphthyl.

The invention particularly relates to the derivatives such as above defined, comprising at least 5, at least 7, at least 8, at least 9 or 10 heterocyclic moieties.

According to another aspect, the invention also particularly relates to the derivatives such as above defined, further comprising one or several, such as two, three or four, 1,3-phenylene-diyl moieties.

Particularly preferred derivatives of the invention have one of the following structures:
- (Het-4 substituted by R2) - (Het-1) - (Het-4 substituted by R2)
- (Het-3 substituted by R1) -(Het-2)- (Het-3substituted by R1)
- (Het-3 substituted by R1) -(Het-2) -(Het-1) -(Het-2)- (Het-3 substituted by R1)
- (Het-3 substituted by R1) -(Het-2)-1,3-phenylene-diyl- (Het-2)-((Het-3 substituted by R1)
- (1,3-phenylene-diyl- substituted by R1 or R2) -(Het-2) -(Het-1) -(Het-2) -(1,3-phenylene-diyl- substituted by R1 or R2)
- (Het-3 substituted by R1) -(Het-2)-(Het-1) (Het-2)-((Het-3 substituted by R1)
- (Het-3 substituted by R1) -(Het-2)-(Het-1)- (Het-1) -(Het-2)-((Het-3 substituted by R1).

In preferred derivatives,
R1 and R2 are oxazolin- or pyridin-diyl groups, and/or
Het-2 is an oxazolin-diyl group and/or
Het-1 is a 2,6 pyridin-diyl, 2,6 pyrimidin-diyl, 2,6 pyrazin-diyl, or 1,3-phenylene-diyl-group.

In an advantageous group, in view of the G-quadruplex interacting properties of the derivatives, Het-1 is linked to two Het-2.

In preferred derivatives of said group, Het-1 is a pyridine-diyl and Het-4 is an oxazolyl, substituted by a pyridyl or a pyridyl-oxazolyl.

In a second advantageous group, Het-2 is linked to two Het-1.

In preferred derivatives, Het-2 is an oxazolyl and Het-1 is a pyridyl oxazolyl.

Specific preferred compounds according to the invention are those listed as the Example section below, especially compounds 3 and 4.

Pharmaceutically acceptable addition salts of the above defined derivatives include acid addition and base salts (including disalts) thereof.

Suitable acid addition salts are formed from acids which form non-toxic salts. Examples include the acetate, aspartate, benzoate, besylate, bicarbonate/carbonate, bisulphate/sulphate, borate, camsylate, citrate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, saccharate, stearate, succinate, tartrate, tosylate and trifluoroacetate salts.

Suitable base salts are formed from bases which form non-toxic salts. Examples include the aluminium, arginine, benzathine, calcium, choline, diethylamine, diolamine, glycine, lysine, magnesium, meglumine, olamine, potassium, sodium, tromethamine and zinc salts.

Such pharmaceutically acceptable salt of an above defined derivative may be readily prepared by mixing together solutions of said derivative and the desired acid or base, as appropriate. The salt may precipitate from solution and be collected by filtration or may be recovered by evaporation of the solvent. The degree of ionisation in the salt may vary from completely ionised to almost non-ionised.

The present invention includes also isotopically-labelled derivatives as above-defined, preferably pharmaceutically acceptable isotopically-labelled derivatives, wherein one or more atoms are replaced by atoms having the same atomic number, but an atomic mass or mass number different from the atomic mass or mass number usually found in nature.

Examples of isotopes suitable for inclusion in the compounds of the invention include isotopes of hydrogen, such as 2H and 3H, carbon, such as 11C, 13C and 14C, chlorine, such as 36C1, fluorine, such as 18F, iodine, such as 1231 and 1251, nitrogen, such as 13N and 15N, oxygen, such as 150, 170 and 180, phosphorus, such as 32P, and sulphur, such as 35S.

Certain isotopically-labelled derivatives according to the invention, for example, those incorporating a radioactive isotope, are useful in drug and/or substrate tissue distribution studies. The radioactive isotopes tritium, i.e. 3H, and carbon-14, i.e. 14C, are particularly useful for this purpose in view of their ease of incorporation and ready means of detection.

Substitution with heavier isotopes such as deuterium, i.e. 2H, may afford certain therapeutic advantages resulting from greater metabolic stability, for example, increased in vivo half-life or reduced dosage requirements, and hence may be preferred in some circumstances.

Substitution with positron emitting isotopes, such as 11C, 18F, 150 and 13N, can be useful in Positron Emission Topography (PET) studies for examining substrate receptor occupancy.

As shown in the Examples hereinafter, the derivatives of the invention specifically bind and stabilise quadruplex DNA and therefore are of great interest as quadruplex-specific probes. The ability of said derivatives to bind to quadruplex DNA as well as their selectivity may be measured using assays know to those skilled in the art, including the assays described in the Example section below.

They are also of great value for the treatment of various diseases.

The invention also relates to pharmaceutical compositions comprising an effective amount of at least one derivative such as above defined in combination with a pharmaceutically acceptable carrier.

As used herein, "therapeutically efficient amount" or "efficient amount" is intended an amount of therapeutic agent such as an above-defined derivative administered to a patient that is sufficient to constitute a treatment of a disease.

As used herein, the term "treatment" of a disease refers to any act aimed at (1) slowing down or stopping the progression, aggravation, re-occurrence, dissemination or deterioration of the symptoms of the disease state or condition to which such term applies; (2) alleviating or bringing about ameliorations of the symptoms of the disease state or condition to which such term applies; and/or (3) reversing or curing the disease state or condition to which such term applies.

The derivatives of the invention intended for pharmaceutical use may indeed be administered alone or in combination with one or more other derivatives of the invention or in combination with one or more other drugs (or as any combination thereof), especially anti-cancer drugs or anti-infectious drugs. Generally, they will be administered as a formulation in association with one or more pharmaceutically acceptable carrier. The term "carrier" is used herein to describe any ingredient other than the derivative(s) of the invention. The choice of carrier will to a large extent depend on factors such as the particular mode of administration, the effect of the carrier on solubility and stability, and the nature of the dosage form.

Pharmaceutical compositions suitable for the delivery of above-defined derivatives and methods for their preparation will be readily apparent to those skilled in the art. Such compositions and methods for their preparation may be found, for example, in 'Remington's Pharmaceutical Sciences', 19th Edition (Mack Publishing Company, 1995).

The pharmaceutical composition may, for example, be in a form suitable for oral administration as a tablet, capsule, pill, powder, sustained release formulations, solution, suspension; for parenteral adminstration as a solution, suspension or emulsion; for topical administration as an ointment or cream; or for rectal administration as a suppository. The pharmaceutical composition may be in unit dosage forms suitable for single administration of precise dosages.

Suitable pharmaceutical carriers include inert diluents or fillers, water and various organic solvents. The pharmaceutical compositions may, if desired, contain additional ingredients such as flavorings, binders, excipients and the like. Thus for oral administration, tablets containing various excipients, such as citric acid may be employed together with various disintegrants such as starch, alginic acid and certain complex silicates and with binding agents such as sucrose, gelatin and acacia. Additionally, lubricating agents such as magnesium stearate, sodium lauryl sulfate and talc are often useful for tableting purposes. Solid compositions of a similar type may also be employed in soft and hard filled gelatin capsules. Preferred materials, therefor, include lactose or milk sugar and high molecular weight polyethylene glycols. When aqueous suspensions or elixirs are desired for oral administration the above-defined derivative may be combined with various sweetening or flavoring agents, coloring matters or dyes and, if desired, emulsifying agents or suspending agents, together with diluents such as water, ethanol, propylene glycol, glycerin, or combinations thereof.

As used herein, "parenteral administration" of a pharmaceutical composition includes any route of administration characterized by physical breaching of a tissue of a subject and administration of the pharmaceutical composition through the breach in the tissue. Parenteral administration thus includes, but is not limited to, administration of a pharmaceutical composition by injection of the composition, by application of the composition through a surgical incision, by application of the composition through a tissue-penetrating non-surgical wound, and the like. In particular, parenteral administration is contemplated to include, but is not limited to, subcutaneous, intravenous, intradermal, intraperitoneal, intramuscular, intrastemal injection, and kidney dialytic infusion techniques.

Formulations of a pharmaceutical composition suitable for parenteral administration comprise the active ingredient combined with a pharmaceutically acceptable carrier, such as sterile water or sterile isotonic saline. Such formulations may be prepared, packaged, or sold in a form suitable for bolus administration or for continuous administration. Formulations for parenteral administration include, but are not limited to, suspensions, solutions, emulsions in oily or aqueous vehicles, pastes, and implantable sustained-release or biodegradable formulations as discussed below. Such formulations may further comprise one or more additional ingredients including, but not limited to, suspending, stabilizing, or dispersing agents. In one embodiment of a formulation for parenteral administration, the active ingredient is provided in dry (i.e. powder or granular) form for reconstitution with a suitable vehicle (e.g. sterile pyrogen-free water) prior to parenteral administration of the reconstituted composition.

The pharmaceutical compositions of the invention can be prepared with carriers that protect the compound against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid.

For therapeutic uses an effective daily amount of active principle in the compositions will in general, be from 0.01 mg/kg to 50 mg/kg body weight, more preferably from 0.1 mg/kg to 10 mg/kg body weight. It will, of course, vary with the derivative employed, the mode of administration, the treatment desired, the disorder indicated as well as with physiological data of the patient (e.g. age, size, and weight). The total daily dose may be administered in single or divided doses. Determining appropriate dosages and regiments for administration of the therapeutic agents is are well-known in the relevant art and would be understood to be encompassed by the skilled artisan.

The above-defined derivatives and pharmaceutical compositions thereof are particularly useful to treat cancer and infectious diseases such as malaria.

The term "cancer", as used herein, refers to the presence of cells possessing characteristics typical of cancer-causing cells, such as uncontrolled proliferation, immortality, metastatic potential, and certain characteristic morphological features. This term refers to any type of malignancy (primary or metastases).

The term « infectious disease » as used herein, refers to a disease resulting from the infection of a mammal (human or animal) by micro-organisms such as bacteria, parasites, yeasts, fungi. Infections by a virus are also included.

The use of the above derivatives and pharmaceutical compositions thereof for making a drug for treating cancer or infectious diseases such as malaria enters into the scope of the invention.

The invention also covers a method for making the above defined derivatives.

Said method comprises
- reacting X1-A-X2
- with B-X3 or with X4-B-X3
   wherein
   - A is di- or tri- or tetra- heteroaryl-1,4 chain and B is a (mono- or di- or tri- or tetra- heteroaryl-1,4)-, the heteroaryl moieties optionally comprising one, two, three or four Aryl-5 moities or Aryl-5 terminal moieties

Heteroaryl-1,4 being a combination of Het-1 and/or Het-2 and/or Het-3 and/or Het-4 which are as above defined,
- X1 and X2, identical or different, represent an hydrogen or halogen or triflate group,
- X3 represents an halogen or zinc halogenide or trialkyltin or boronate group
- -X4 represents a carboxaldehyde group,
   under conditions giving the desired polyheteroaryl derivatives.

Other characteristics and advantages of the invention are given in the following Examples, which refer to Fig.1 to Fig. 16, wherein
- Fig.1A- Fig.1D represents absorption spectra of a derivative according to the invention in various conditions;
- Fig.2, various titrations summary;
- Fig.3A to Fig.3C, results of FRET-melting assays with oligonucleotides mimicking the human or plasmodium telomeric sequences and FRET partners;
- Fig.4A-Fig.4b and Fig.5, results of fluorescence melting of G-quadruplex with or without a derivative of the invention;
- Fig.5, melting fluorescence results of a G-quadruplex alone or in the presence of a derivative of the invention, without or with excess of quadruplex-DNA competitors;
- Fig.6, the summary of the various FRET-melting experiments;
- Fig.7A and Fig.7B, results of fluorescence melting with a quadruplex-DNA with addition of a derivative of the invention;
- Fig.8, fluorescence titration results when using a quadruplex or using a short-duplex DNA with addition of a derivative of the invention;
- Fig.9, fluorescence titration results when using various quadruplex-DNA structures with addition of a derivative of the invention;
- Fig.10, a graphical representation of the summary of the various fluorescence titrations;
- Fig. 11A and Fig.11B and Fig.12, results of circular dichroism of quadruplex-DNA with addition of a derivative of the invention;
- Fig. 13, results relating to quadruplex-structure induction;
- Fig. 14, results relating to the inhibition of POT1 binding to telomeric sequence *in vitro* by a derivative according to the invention;
- Fig.15, results relating to the inhibition of cell proliferation by a derivative according to the invention, and
- Fig.16, results relating to the stabilisation of quadruplex-DNA and quadruplex-over duplex-DNA selectivity for derivatives of the invention.

### I - Synthesis of Poly-heteroaryl derivatives

The following examples are intended to illustrate the present invention. The compounds may also be obtained by other processes known by the one skilled in the art.
2,6-pyridine dicarboxaldehyde, 6-bromopyridine-2-carbaldehyde, *p*-toluenesulfonylmethyl isocyanide (TosMIC), 2-pyridylzinc-bromide, 1,3-benzene dicarboxaldehyde, 6-bromopyridine-2-carboxaldehyde, 3-bromobenzaldehyde and 5-bromo-2-furaldehyde used as starting materials, are commercially available products.

### 1. : Example 1: 2,6-Bis[2-(pyridin-2-yl)oxazol-5-yl]pyridine (Compound 1)

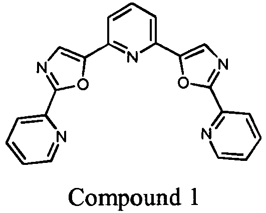

### 1.1.: 2,6-Bis(oxazol-5-yl)pyridine (intermediate 1)

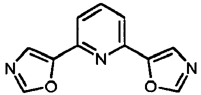

2,6-Pyridine dicarboxaldehyde (4.0 g ; 29.2 mmol), TosMIC (11.4 g ; 58.3 mmol) and potassium carbonate (16. 3 g ; 117.8 mmol) in 100 mL of methanol were heated at reflux during 3 hours. The solvent was evaporated in vacuo and the residue was poured into brine solution and extracted with dichloromethane (4x 150 mL). The combined organic layers were dried over magnesium sulfate and concentrated in vacuo. The residue was purified by flash chromatography on silica gel column with dichloromethane-ethanol (95-5) as the eluent to give the titled compound as yellow solid (5.6 g, 90%), m.p.= 190-191°C ; ¹H NMR (300 MHz, CDCl3) 7.56 (d ; 2H ; J= 8.0) ; 7.74 (s ; 2H) ; 7.80 (t ; 1H ; J= 8.0 Hz) ; 7.96 (s ; 2H) ; 13
C NMR (75 MHz, CDCl3) 118.7, 125.7, 138.0, 147.4, 150.8, 151.0; SM m/z 214.1 (M+1) ; Anal. calcd for C₁₁H₇N₃O₂ : C, 91.97 ; H, 3.31 ; N, 19.71. found: C, 61.51, H, 3.43, 19.48.

### 1.2.: 2,6-Bis-(2-iodooxazol-5-yl)pyridine (intermediate 2)

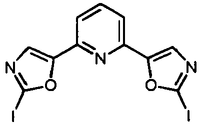

Under an argon atmosphere, the intermediate 1 (0.1 g ; 0.5 mmol) and TMEDA (0.2 mL ; 1.0 mmol) are dissolved in 5 mL of anhydrous THF and cooled at -78°C. A solution of LiHMDS 1M in THF (0.5 mL ; 0.5 mmol) was added dropwise and stirred during 30 minutes at -78°C and one hour at -40°C. The mixture was cooled again at -78°C and 1,2-diiodoethane (0.5 g ; 1.9 mmol) was added. After stirring over night at room temperature the mixture was poured into a sodium thiosulfate solution and extracted with ethyl acetate (3x10 mL). The combined organic layers were dried over magnesium sulfate and concentrated in vacuo. The residue was purified by flash chromatography (SiO₂, dichloromethane-ethanol, 95-5) to give the intermediate 2 as beige solid (117 mg, 50%), ¹H NMR (300 MHz, CDCl3) 7.58 (d ; 2H ; 13 J= 8 Hz) ; 7.69 (s ; 2H) ; 7.85 (t ; 1H ; J= 8 Hz) ; C NMR (75 MHz, CDCl3) 119.5, 129.7, 138.7, 147.0, 157.2, 151.5; SM *m*/*z* 465.8 (M+1), 487.8 (M+23).

### 1.3.: 2,6-bis[2-(pyridin-2-yl)oxazol-5-yl]pyridine (Compound 1)

Method 1 : A mixture of intermediate 2 (65 mg ; 0.14 mmol), 2-pyridylzinc-bromide (1.2 mL ; 0.60 mmol) and Pd(PPh₃)₄ (12 mg ; 0.01 mmol) were heated at reflux in 2 mL of anhydrous THF during 4 hours. Water was added and the mixture was extracted with ethyl acetate (3x10 mL). The combined organic layers were dried over magnesium sulfate, filtered and concentrated *in vacuo.* The residue was purified by flash chromatography (SiO₂, dichloromethane-ethanol, 95-5) to give the expected compound 1 as beige solid (13 mg, 25%), m.p.=242-245°C ; ¹H NMR (300 MHz, CDCl3) 7.40 (m ; 2H) ; 7.83-7.88 (m ; 5H) ; 13 7.97 (s ; 2H) ; 8.23 (d ; 2H ; J= 7.9 Hz) ; 8.77 (d ; 2H ; J= 4.5 Hz) ; C NMR (75 MHz, CDCl3) 119.7, 123.3, 125.6, 128.6, 137.8, 138.5, 146.6, 147.9, 150.9,152.3, 161.5; SM *m*/*z* 368.0 (M+1), 390.0 (M+23).

Method 2 : A mixture of intermediate 1 (0.1 g ; 0.5 mmol), 2-bromopyridine (91 µL ; 0.9 mmol), palladium diacetate (11 mg ; 0.05 mmol ; 10 mol%), PCy₃.HBF₄ (35 mg ; 0.1 mmol ; 20 mol%), copper (I) iodide (0.18 g ; 0.9 mmol), cesium carbonate (0.61 mg ; 1.88 mmol) and 1.3 mL of anhydrous toluene were submitted under microwave irradiation conditions (130°C, 150W) during 4 hours. The residue was purified by flash chromatography (SiO₂, dichloromethane-ethanol, 95-5) to give the compound 1 (8 mg, 33%) which is identically with than that described in method 1 above.

### 2. Example 2 : 2,5-Bis-[6-(oxazol-5-yl)]pyridin-2yloxazole (Compound 2)

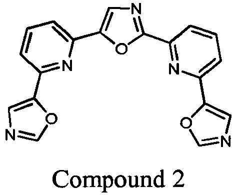

### 2.1: 5-(2-Bromopyridin-2 yl)oxazole (intermediate 3)

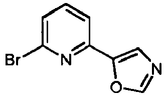

6-Bromopyridine-2-carbaldehyde (4.0 g ; 21.5 mmol), TosMIC (4.2 g ; 21.5 mmol) and potassium carbonate (6.0 g ; 43.4 mmol) were heated at reflux during 3 hours in 75 mL of methanol. The solvent was evaporated in vacuo and the residue was poured into brine solution and extracted with dichloromethane (4x100 mL). The combined organic layers were dried over magnesium sulfate and concentrated in vacuo. The residue was purified by flash chromatography (SiO₂, dichloromethane-ethanol, 95-5) to give the intermediate 3 as yellow solid (2.9 g, 60%); m.p.= 90-91°C ; ¹H NMR (300 MHz, CDCl₃) 7.38 (dd ; 1H ; J= 3.5 & 5.3 13 Hz) ; 7.57 (m ; 2H) ; 7.73 (s ; 1H) ; 7.97 (s ; 1H) ; C NMR (75 MHz, CDCl3) 118.1, 126.4, 127.6, 139.3, 142.4, 148.0, 151.6 ; SM *m*/*z* 225.0 and 227.1 (M+1).

### 2.2: 2,5-Bis-[6-(oxazol-5-yl)]pyridin-2yloxazole (Compound 2)

A mixture of intermediate 1 (0.10 g ; 0.5 mmol), intermediate 3 (0.10 g ; 0.5 mmol), palladium diacetate (11 mg ; 0.05 mmol ; 10 mol%), PCy₃.HBF₄ (35 mg ; 0.09 mmol ; 20 mol%), copper (I) iodide (0.1 g ; 0.5 mmol), cesium carbonate (0.3 mg ; 0.9 mmol) and 1.3 mL of anhydrous toluene were submitted under microwave irradiation conditions (130°C, 150W) during 6 hours. The residue was purified by flash chromatography (SiO₂, dichloromethane-ethanol, 95-5) to give the expected compound 2 as beige solid (60 mg, 36%); ¹H NMR (300 MHz, CDCl₃) 7.70 (d ; 2H ; J= 7.5 Hz) ; 7.80 (d ; 1H; J= 7.5Hz) ; 7.85 (d ; 2H ; J=7.5Hz) ; 7.90 (s ; 1H) ; 7.93 (d; 2H ; J= 5 Hz) ; 8.00 (s ; 1H) ; 8.04 (d ; 1H ; J= 5 Hz) ; 8.20 (d ; 1H ; J= 7.5 Hz); SM *m*/*z*358.1 (M+1).

### 3. Example 3 : 2,6-bis{2-[6-(oxazol-5-yl)pyridin-2-yl]oxazol-5-yl}pyridine (Compound 3).

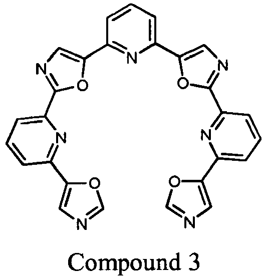

A mixture of intermediate 1 (0.10 g ; 0.5 mmol), intermediate 3 (0.21 g ; 0.9 mmol), palladium diacetate (11 mg ; 0.05 mmoles ; 10 mol%), PCy₃.HBF₄ (35 mg ; 0.09 mmol ; 20 mol%), copper (I) iodide (0.18 g ; 0.9 mmol), cesium carbonate (0.61 mg ; 1.9 mmol) and 1.3 mL of anhydrous toluene were submitted under microwave irradiation conditions (130°C, 150W) during 6 hours. The residue was purified by flash chromatography (SiO₂, dichloromethane-ethanol, 95-5) to give the expected compound 3 as beige solid (52 mg, 22%) m.p. >250°C ; ¹H NMR (300 MHz, CDCl3) 7.65 (m; 1H) ; 7.75 (m; 2H); 7.90-8.05 (m; 9H); 8.10 (s; 1H); 8.20 (m; 2H); SM *mlz* 524.2 (M+23).

### 4. Example 4 : 1,3-Bis(2-(6-(oxazol-5-yl)pyridin-2-yl)oxazol-5-yl)benzene (Compound 4).

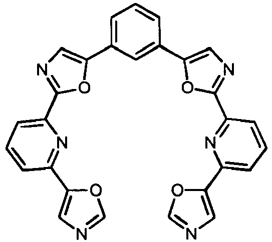

### Compound 4

### 4.1: 6,6'-(5,5'-(1,3-Phenylene)bis(oxazole-5,2-diyl))dipyridine-2-carbaldehyde (intermediate 4)

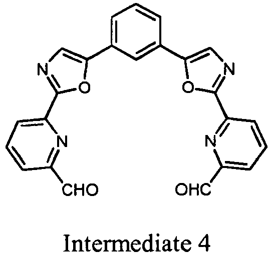

A mixture of 1,3-di(oxazol-5-yl)benzene, obtained as described by Sambavisarao and Vrajesh (Synthsis, 2007, 3653), (0.21 g ; 1 mmol), 6-bromopyridine-2-carboxaldehyde (0.24 g ; 1.3 mmol; palladium diacetate (64 mg ; 0.28 mmol, 28 mol%), PCy₃.HBF₄ (62 mg ; 0.17 mmol; 17 mol%), copper (I) iodide (0.43 g ; 2.3 mmol), cesium carbonate (1.4 g ; 4.3 mmol) and 4 mL of anhydrous dioxane were heated in a sealed tube at 130°C during 2 hours. The residue was purified by flash chromatography (SiO₂, dichloromethane-ethanol, 95-5) to give the intermediate 4 as beige solid (83 mg, 30%); ¹H NMR (300 MHz, CDCl₃) 10.27 (s ; 1H); 8.45-8.40 (m ; 1H) ; 8.20 (s ; 0.5H) ; 8.10-8.05 (m ; 2H) ; 7.83 (dd ; 1H; J= 7.8 & 1.6 Hz) ; 7.71 (s; 1H) ; 7.61 (t ; 0.5H; J= 7.93 Hz); SM *m*/*z* 423.1 (M+1).

### 4.2 : 1,3-Bis(2-(6-(oxazol-5-yl)pyridin-2-yl)oxazol-5-yl)benzene (Compound 4).

A mixture of dicarboxaldehyde intermediate 4 (75 mg ; 0.18 mmol), TosMIC (86 mg ; 0.44 mmol) and potassium carbonate (126 mg ; 0.9 mmol) in 8 mL of absolute ethanol was heated at reflux during 2 hours. The solvent was evaporated in vacuo and the residue was poured into brine solution and extracted with dichloromethane (3x50 mL). The combined organic layers were dried over magnesium sulfate and concentrated in vacuo. The residue was purified by flash chromatography on silica gel column with dichloromethane-ethanol (95-5) as the eluent to give the titled compound as off-white solid (55 mg, 62%), m.p.> 250°C ; ¹H NMR (300 MHz, CDCl₃) 8.22 (br s ; 0.5H) ; 8.17 (d ; 1H; J= 7.8 Hz) ; 8.03 (s; 1H) ; 7.99 (t; 1H, J= 7.8 Hz), 7.93 (s; 1H) ; 7.85-7.76 (m; 2H); 7.68 (s; 1H); 7.64-7.57 (m; 0.5H); SM *mlz* 501.1 (M+1) ; Anal. calcd for C₂₈H₁₆N₆O₄·0.75 H₂O: C, 65.43 ; H, 3.40 ; N, 16.36, found: C, 65.92, H, 3.37, 15.92.

### 5. Example 5 : 2,6-Bis(2-(3-(oxazol-5-yl)phenyl)oxazol-5-yl)pyridine (Compound 5).

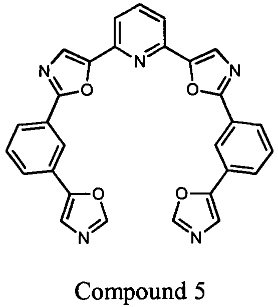

### 5.1 : 3,3'-(5,5'-(Pyridine-2,6-diyl)bis(oxazole-5,2-diyl))dibenzaldehyde (intermediate 5)

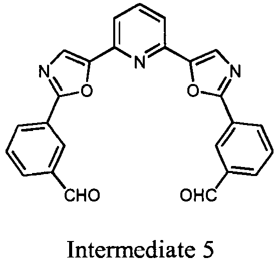

A mixture of intermediate 1 (0.18 g ; 0.84 mmol), 3-bromobenzaldehyde (0.50 g ; 2.7 mmol), palladium diacetate (80 mg ; 0.36 mmol ; 43 mol%), PCy₃.HBF₄ (68 mg ; 0.18 mmol ; 20 mol%), copper (I) iodide (0.37 g ; 1.9 mmol), cesium carbonate (1.2 g ; 3.7 mmol) and 3.5 mL of anhydrous dioxane were heated in a sealed tube at 130°C during 18 hours. The residue was purified by flash chromatography (SiO₂, dichloromethane-ethanol, 98-2) to give the intermediate 5 as yellow solid (50 mg, 14%); ¹H NMR (300 MHz, CDCl₃) 10.15 (s ; 1H); 8.66 (s ; 1H) ; 8.45 (d ; 1H; J= 7.7 Hz) ; 8.04 (d; 1H; J= 7.6 Hz) ; 7.98-7.92 (m ; 1.5H) ; 7.76 (d ; 1H; J= 7.9 Hz) ; 7.72 (t ; 1H; J= 7.7 Hz); SM *m*/*z* 444.0 (M+Na); Anal. calcd for C₂₅H₁₅N₃O₄· 0.25 H₂O: C, 70.50 ; H, 3.64 ; N, 9.87, found: C, 70.11, H, 3.51, 9.75.

### 5.2 : 2,6-Bis(2-(3-(oxazol-5-yl)phenyl)oxazol-5-yl)pyridine (Compound 5).

A mixture of dicarboxaldehyde intermediate 5 (50 mg ; 0.12 mmol), TosMIC (50 mg ; 0.25 mmol) and potassium carbonate (70 mg ; 0.5 mmol) in 6 mL of absolute ethanol was heated at reflux during 2 hours. The solvent was evaporated in vacuo and the residue was poured into brine solution and extracted with dichloromethane (3x50 mL). The combined organic layers were dried over magnesium sulfate and concentrated in vacuo. The residue was purified by flash chromatography on silica gel column with dichloromethane-ethanol (94-6) as the eluent to give the titled compound as pale yellow solid (40 mg, 67%), m.p. 254-256°C ; ¹H NMR (300 MHz, CDCl₃) 8.45 (s ; 1H) ; 8.14 (d ; 1H; J= 7.9 Hz) ; 7.99 (s; 1H) ; 7.96-7.88 (m; 1.5H), 7.79 (d ; 1H; J= 7.9 Hz); 7.74 (d ; 1H; J= 7.9 Hz); 7.60 (t; 1H; J= 7.9 Hz); 7.51 (s; 1H); SM m/z 500.2 (M+1) ; Anal. calcd for C₂₉H₁₇N₅O₄· H₂O: C, 67.31 ; H, 3.67 ; N, 13.53, found: C, 67.53, H, 3.82, 13.29.

### 6. Example 6 : 2,6-Bis(2-(5-(oxazol-5-yl)furan-2-yl)oxazol-5-yl)pyridine (Compound 6).

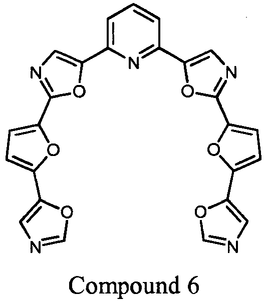

### 6.1 : 5,5'-(5,5'-(Pyridine-2,6-diyl)bis(oxazole-5,2-diyl))difuran-2-carbaldehyde (intermediate 6).

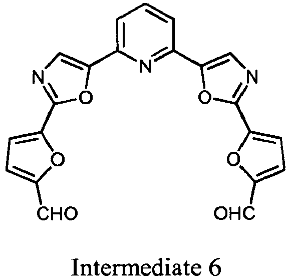

A mixture of intermediate 1 (0.21 g ; 1 mmol), 5-bromo-2-furaldehyde (0.21 g ; 1.2 mmol), palladium diacetate (83 mg ; 0.37 mmol ; 37 mol%), PCy₃.HBF₄ (72 mg ; 0.2 mmol ; 20 mol%), copper (I) iodide (0.46 g ; 2.4 mmol), cesium carbonate (1.4 g ; 4.3 mmol) and 4 mL of anhydrous dioxane were heated in a sealed tube at 130°C during 2 hours. The residue was purified by flash chromatography (SiO₂, dichloromethane-ethanol, 98-2) to give the intermediate 6 as yellow solid (62 mg, 26%); ¹H NMR (300 MHz, CDCl₃) 9.83 (s ; 1H); 7.97 (s ; 1H) ; 7.95-7.90 (m ; 0.5H) ; 7.79 (d; 1H; J= 8.2 Hz) ; 7.39 (d; 1H; J= 3.8 Hz) ; 7.31 (d ; 1H; J= 3.8 Hz); SM *m*/*z* 402.1 (M+1); Anal. calcd for C₂₁H₁₁N₃O₆· 1.5 H₂O: C, 58.87 ; H, 3.27 ; N, 9.81, found: C, 58.77, H, 2.85, 9.83.

### 6.2 : 2,6-Bis(2-(5-(oxazol-5-yl)furan-2-yl)oxazol-5-yl)pyridine (Compound 6).

A mixture of dicarboxaldehyde intermediate 6 (30 mg ; 0.07 mmol), TosMIC (43 mg ; 0.22 mmol) and potassium carbonate (63 mg ; 0.45 mmol) in 3 mL of absolute ethanol was heated at reflux during 2 hours. The solvent was evaporated in vacuo and the residue was poured into brine solution and extracted with dichloromethane (3x50 mL). The combined organic layers were dried over magnesium sulfate and concentrated in vacuo. The residue was purified by flash chromatography on silica gel column with dichloromethane-ethanol (96-4) as the eluent to give the titled compound as pale yellow solid (13 mg, 36%), m.p.> 250°C ; ¹H NMR (300 MHz, CDCl₃) 7.95-7.91 (m; 2.5H) ; 7.71 (d ; 1H; J= 8.1 Hz) ; 7.53 (s; 1H) ; 7.34-7.29 (m; 2H); SM *m*/*z* 480.1 (M+1).

### 7. : 2,6-Bis(2-(2,2'-bipyridin-6-yl)oxazol-5-yl)pyridine (Compound 7).

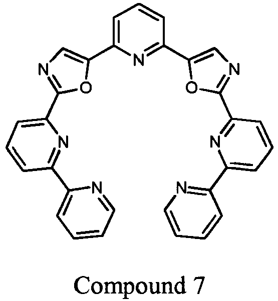

The protocole described above for the synthesis of compound 3 is applied, starting from the intermediate 1 and using 6-bromo-2,2'-bipyridine (U. Lehmann and A.D. Schlüter, Eur. J. Org. Chem. 2000, 3483-3487) in place of intermediate 3, to give the title compound 7.

### 8. 2,6-Bis(2-(6-(oxazol-5-yl)pyridin-2-yl)oxazol-5-yl)pyrazine (Compound 8).

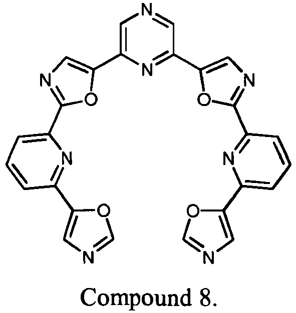

### 8.1 : 2,6-Di(oxazol-5-yl)pyrazine (intermediate 7).

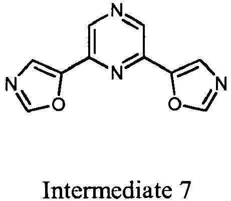

The protocole described above for the synthesis of intermediate 1 is applied, starting from pyrazine-2,6-dicarbaldehyde (H. Schumann and H.-K. Luo, Zeitschrift fuer Naturforschung, B : Chemical Sciences, 2005, 60(1), 22-24) in place of 2 ,6-pyridine dicarboxaldehyde, to give the title intermediate 7.

### 8.2 : 6,6'-(5,5'-(Pyrazine-2,6-diyl)bis(oxazole-5,2-diyl))dipyridine-2-carbaldehyde (intermediate 8).

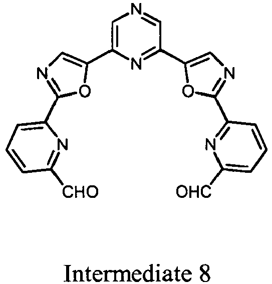

The protocole described above for the synthesis of intermediate 5 is applied, starting from intermediate 7 and 6-bromopyridine-2-carbaldehyde in place of intermediate 1 and 3-bromobenzaldehyde, to give the title intermediate 8.

### 8.3 : 2,6-Bis(2-(6-(oxazol-5-yl)pyridin-2-yl)oxazol-5-yl)pyrazine (Compound 8).

The protocole described above for the synthesis of compound 5 is applied, starting from intermediate 8 in place of intermediate 5, to give the title compound 8.

### 9. : 6.6'-Bis(2-(6-(oxazol-5-yl)pyridin-2-yl)oxazol-5-yl)-2,2'-bipyridine (Compound 9).

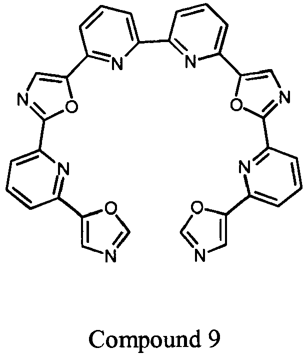

### 9.1 : 6,6'-Di(oxazol-5-yl)-2,2'-bipyridine (intermediate 10).

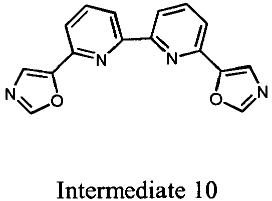

The protocole described above for the synthesis of intermediate 1 is applied, starting from 2,2'-bipyridine-6,6'-dicarbaldehyde (G. R. Newkome and H.-W. Lee; J. Am. Chem. Soc. 1983, 105(18), 5956-5957) in place of 2 ,6-pyridine dicarboxaldehyde, to give the title intermediate 10.

### 9.2 : 6,6'-(5,5'-(2,2'-Bipyridine-6,6'-diyl)bis(oxazole-5,2-diyl))dipyridine-2-carbaldehyde (intermediate 11).

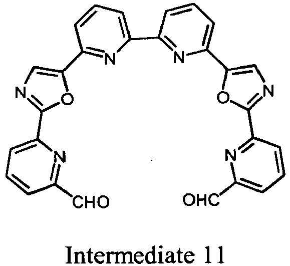

The protocole described above for the synthesis of intermediate 5 is applied, starting from intermediate 10 and 6-bromopyridine-2-carbaldehyde in place of intermediate 1 and 3-bromobenzaldehyde, to give the title intermediate 11.

### 9.3 : 6,6'-Bis(2-(6-(oxazol-5-yl)pyridin-2-yl)oxazol-5-yl)-2,2'-bipyridine (Compound 9).

The protocole described above for the synthesis of compound 5 is applied, starting from intermediate 11 in place of intermediate 5, to give the title compound 9.

### II-Biophysical methods and data:

### Study II-1: UV-vis spectroscopy for investigating solubility properties

### a) Uv-vis titrations:

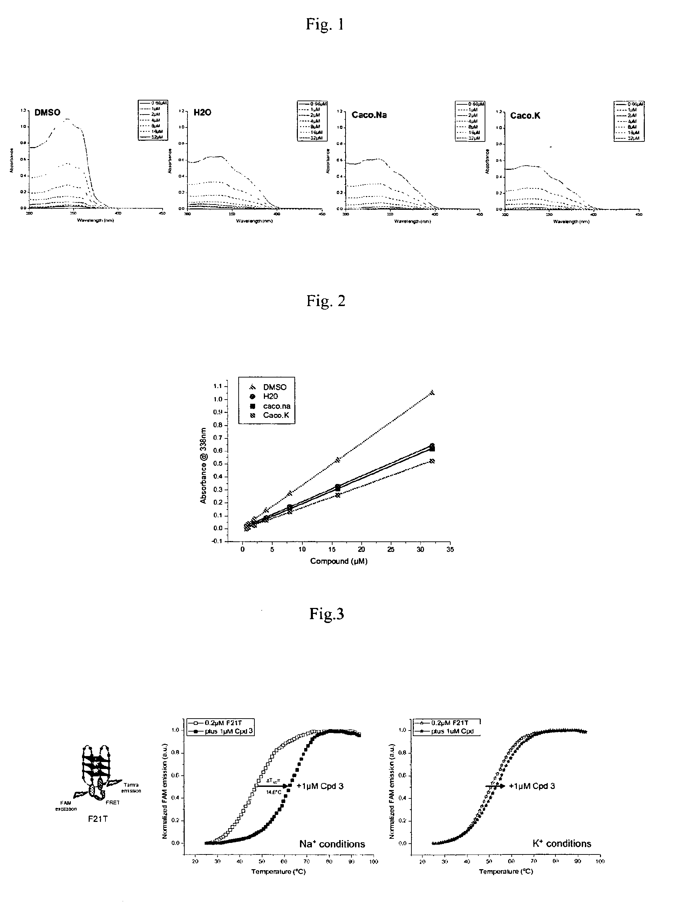

The study of UV-vis properties of pyridine-based polyheteroaromatic compounds detailed in this study enable the determination of their solubility in various conditions (DMSO, H₂O and cacodylate buffers (10mM sodium cacodylate + 100mM NaCl (for Caco.Na) or KCl (for Caco.K)): the simplest method for this is to measure the absorption spectra of compound 3 at various concentrations (from 0 to 32µM); solubility of compound 3 is thus evaluated through the reporting of its absorbance at a given wavelength (herein 338nm (A₃₃₈)) as a function of the concentration of compound 3 and applying the Beer-Lambert law. The results are given in Fig.1.

### b) Summary

The various titrations are summarized in Fig.2

These data indicated that compound 3:
- is soluble in all the solvent systems used herein; (DMSO, H₂O and aqueous cacodylate buffers (both Na⁺- or K⁺-rich);
- does not self-aggregate within the 0 to 32µM concentration range, in all the solvent systems used herein.

### Study II-2: Stabilization of quadruplex-DNA and quadruplex- over duplex-DNA selectivity

Stabilization, and so interaction, of compounds with quadruplex-structure is monitored *via* FRET-melting assay, in a version that also enables the determination of the quadruplex-over duplex-DNA selectivity as well as the intra-quadruplex selectivity (17).

FRET assay is performed with oligonucleotides that mimic the human or plasmodium telomeric sequences, and equipped with FRET partners at each extremities: F21T (*FAM-*G₃[T₂AG₃]₃-*Tamra*,) FPf1T (*FAM*-G₃[T₃AG₃]₃-*Tamra*) and FPf8T (*FAM*-G₃[T₂CAG₃]₃-*Tamra*) with *FAM*: 6-carboxyfluorescein and *Tamra*: 6-carboxy-tetramehtylrhodamine). Measurements were made with excitation at 492nm and detection at 516nm.

### a) Quadruplex-stabilization:

Fluorescence melting were carried out. The results are given on Fig.3A- Fig.3C. The experiments were carried out with 0.2µM of F21T, FPf1T or FPf8T in a buffer containing 10mM lithium cacodylate pH 7.2 and 100mM NaCl or KCl (see example of F21T below, NaCl (left) and KCl (right)); the melting of the quadruplex-DNA was monitored alone and in the presence of 1µM (5 equiv.) of compound 3.

The stabilisation effect induced by compound 3 with the three oligonucleotides is quantified by the increase in melting temperature (ΔT_{1/2}). The values are indicated in the table below.

| ΔT_{1/2} (°C) | F21T | FPf1T | FPf8T |
|---|---|---|---|
| Na⁺ | 14,6 | 20,4 | 16,3 |
| K⁺ | 1,3 | 2,1 | 1,5 |

### b) Quadruplex- vs duplex-DNA selectivity:

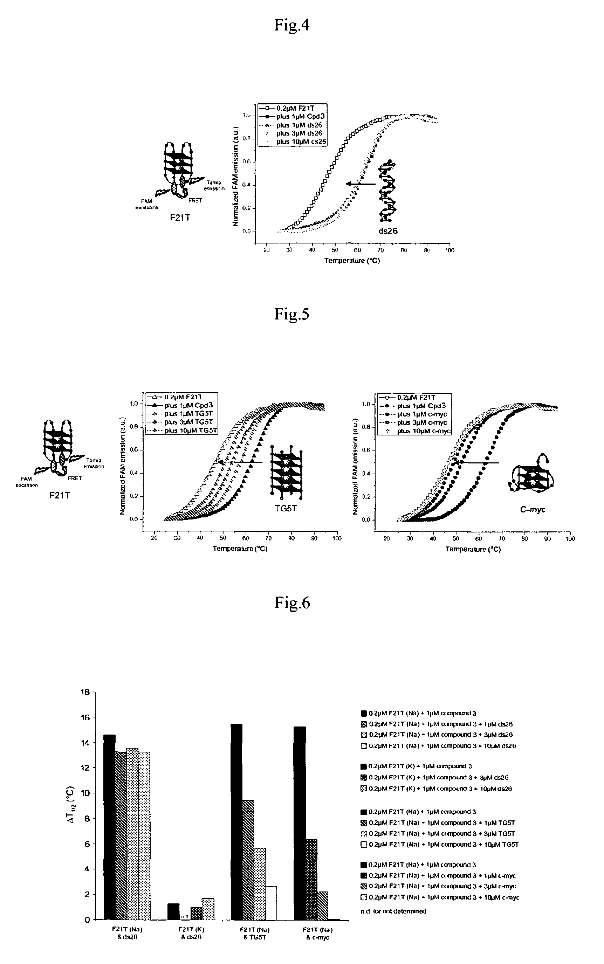

Fluorescence melting are carried out with 0.2µM of F21T in a buffer containing 10mM lithium cacodylate pH 7.2 and 100mM NaCl; the melting of the G-quadruplex was monitored alone and in the presence of 1µM of compound 3 without or with excess (1, 3 and 10µM) of duplex-DNA competitor ds26 (a 26 base-pair duplex-DNA comprised of the self complementary sequence [5'-CAATCGGATCGAATTCGATCCGATTG-3']). The results are given on Fig. 4

### c) Intra-quadruplex selectivity:

Fluorescence melting experiments are carried out with 0.2µM of F21T in a buffer containing 10mM lithium cacodylate pH 7.2 and 100mM NaCl; the melting of the G-quadruplex was monitored alone and in the presence of 1µM of compound 3 without or with excess (1, 3 and 10µM) of quadruplex-DNA competitors, both TG5T ([(5'-TG₅T-3')₄], a tetramolecular quadruplex-DNA used in previous study (21) or c-myc ([5'-GAGGGTGGGGAGGGTGGGGAAG-3'], a sequence present in the promoter region of the oncogene c-myc, highly suspected to fold into an intramolecular quadruplex-structure (22-25). The results are given on Fig. 5A -Fig.5B.

### d) Summary

The various FRET-melting experiments are summarized in the graphical bar representation of Fig.6.

These data indicate that compound 3:
- interacts efficiently with telomeric quadruplexes F21T (human), FPf1T et FPf8T (*Plasmodium falciparum*) in sodium buffer while it does not stabilize these oligonuclotides in potassium conditions (see Table), meaning that in the three cases examined, compound 3 presents cation-based intra-quadruplex selectivity;
- discerns very efficiently quadruplex- from duplex-DNA, since F21T stabilization (in sodium buffer) is only limitedly affected by the presence of 1, 3 or 10µM of ds26 (the stabilization is >91% maintained in all cases).
- interacts most probably *via* stacking interactions with external G-quartet of quadruplex-structures since the F21T stabilization is highly sensitive to the competition of non-looped tetramolecular quadruplex-DNA (TG5T, F21T stabilization being maintained at 61, 37 and 17% in presence of 1, 3 and 10µM of TG5T respectively) or only-double chain reversal-looped quadruplex-DNA (c-myc, F21T stabilization being maintained at 42, 15 and 1% in presence of 1, 3 and 10µM of c-myc respectively).
- Interaction of the compound both with tetrads and loops can also be hypothesized given the dramatic difference observed between Na⁺ and K⁺ conditions.

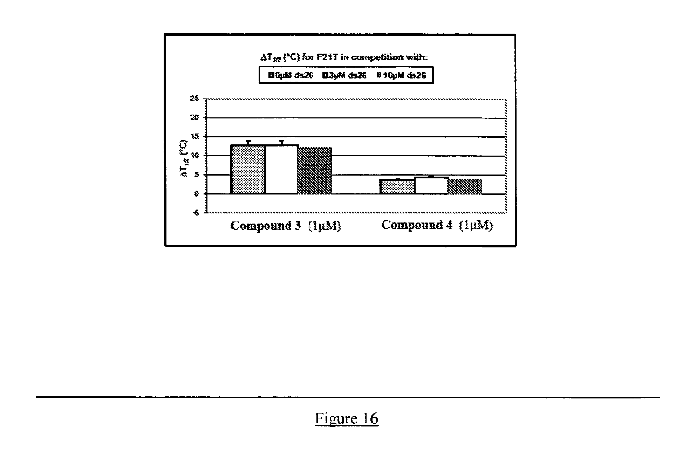

Study II-2bis: Fig.16 gives results obtained in FRET-melting, using the same sodium conditions as above defined in a) and b), with compounds 3 and 4. The melting of the Fluorescently labelled quadruplex F21T was monitored alone and in the presence of 1µM of compound 3 or compound 4 without or with excess (1.3 and 10µM) of duplex-DNA competitor ds26 (a 26 base-pair duplex DNA comprised the above mentioned self-complementary sequence [5'-CAATCGGATCGAATTCGATCCGATTG-3']).

### An increase in melting temperature, poorly affected by the presence of ds26, is obtained for both compounds.

### Study II-3: Interaction with DNA monitored by fluorescence studies

Pyridine-based polyheteroaromatic compounds detailed in this study are characterized by a strong fluorescence. Remarkably, the quantum yield is not affected by the nature of the solvent they are used in, from pure organic (e.g. DMSO) to physiological conditions (*e.g.* buffer: 10mM sodium cacodylate + 100mM KCl, pH 7.2, see Table). The modification of the fluorescence properties of studied compounds upon interaction with DNA enables to compare their apparent binding affinity for several quadruplexes of biological relevance (27).

| | DCM | Water |
|---|---|---|
| Quantum yield | 50.35% | 50.37% |
| s.d. | 2.17% | 2,89% |

*NB:* the quantum yields (and standard deviations (s.d.)) of compound 3 were measured in CH₂Cl₂ (DCM) and water, with anthracene in ethanol as reference.

### a) Quadruplex-interaction:

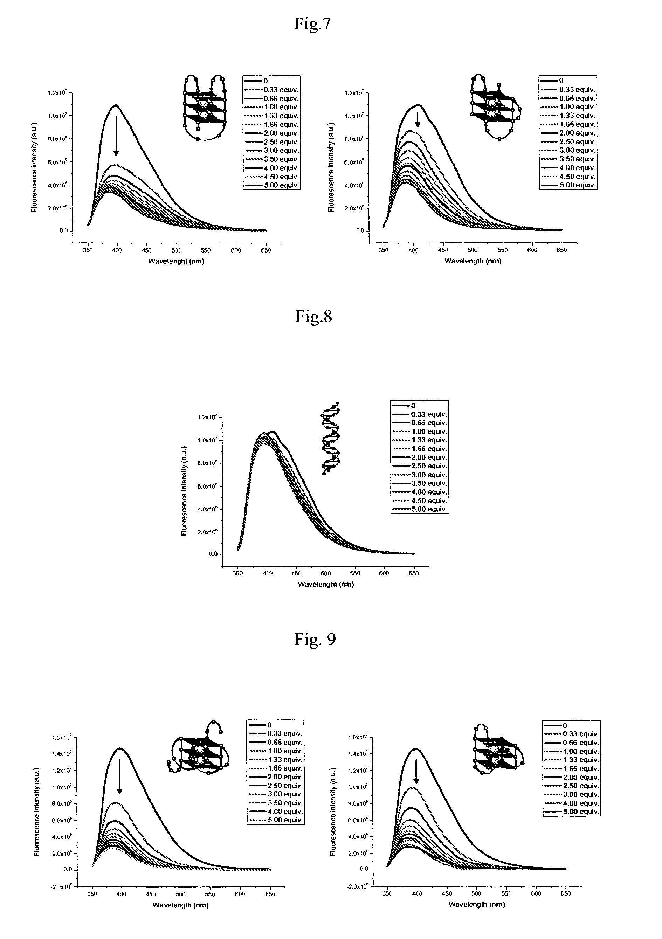

The quadruplex-DNA used herein is 22AG: it results from the folding of a 22nt oligonucleotide that mimics the human telomeric sequence: 22AG is [5'-AG₃(T₂AG₃)₃-3']. Quadruplex-structure from 22AG is prepared by heating the corresponding oligonucleotide at 90°C for 5min in a 10mM sodium cacodylate buffer pH 7.2, 100 mM NaCl (for 22AG Na) or KCl (for 22AG K) and cooling in ice to favor the intramolecular folding by kinetic trapping. Concentrations are determined by UV-Vis measurements (after thermal denaturation, 5min at 85°C) at 260nm before use. (see Fig 7A and Fig.7B).

Increasing amounts of 22AG Na (left) or 22AG K (right) are added onto a solution of 0.25µM of compound 3 in cacodylate buffer (10mM sodium cacodylate + 100mM NaCl (right) or KCl (left)), which result in a progressive quench of the fluorescence of compound 3 (λₑₓ = 340nm).

### b) Quadruplex- vs duplex-DNA selectivity:

The quadruplex- over duplex-selectivity is evaluated through fluorescence titrations by comparison of experiments carried out with 22AG (see above) and with a short duplex-DNA: ds17, which is a 17 base-pair duplex-DNA that represents a biological sequence used in previous studies (28); the sequences of the two complementary strands are the following: [5'-CCAGTTCGTAGTAACCC-3']/[5'-GGGTTACTACGAACTGG-3']. Duplex-structure is prepared by heating the two corresponding complementary strands at 90°C for 5min in a 10mm sodium cacodylate buffer pH 7.3, 100 mM KCl followed by a slow cooling over 6hrs. Concentrations are determined by UV-Vis measurements (after thermal denaturation, 5min at 85°C) at 260nm before use.

The results are given on Fix.8.

Increasing amounts of ds17 are added onto a solution of 0.25µM of compound 3 in cacodylate buffer (10mM sodium cacodylate + 100mM KCl) do not result in a progressive quench of the fluorescence of compound 3 (λₑₓ = 340nm).

### c) Intra-quadruplex selectivity:

The intra-quadruplex selectivity is evaluated through fluorescence titrations by comparison of experiments carried out with 22AG (see above) and with two other quadruplex-structures: c-myc and c-kit2. The formation of these two quadruplex-DNAs is currently highly suspected in the promoter region of c-myc (see above) and c-kit (22; 29 and 30)oncogenes. The sequences are the following: c-myc: [5'-TGAGGGTGGGTAGGGTGGGTAA-3'] and c-kit2 : [(5'-CGGGCGGGCGCGAGGGAGGGG-3']; Quadruplex-structures are prepared by heating the corresponding oligonucleotide at 90°C for 5min in a 10mM sodium cacodylate buffer pH 7.2, 100 mM KCl and cooling in ice to favor the intramolecular folding by kinetic trapping. Concentrations are determined by UV-Vis measurements (after thermal denaturation, 5min at 85°C) at 260nm before use.

The results are given on Fig.9A and Fig.9B.

Increasing amounts of c-myc (left) or c-kit2 (right) are added onto a solution of 0.25µM of compound 3 in cacodylate buffer (10mM sodium cacodylate + 100mM KCl), which result in a progressive quench of the fluorescence of compound 3 (λₑₓ = 340nm).

### d) Summary:

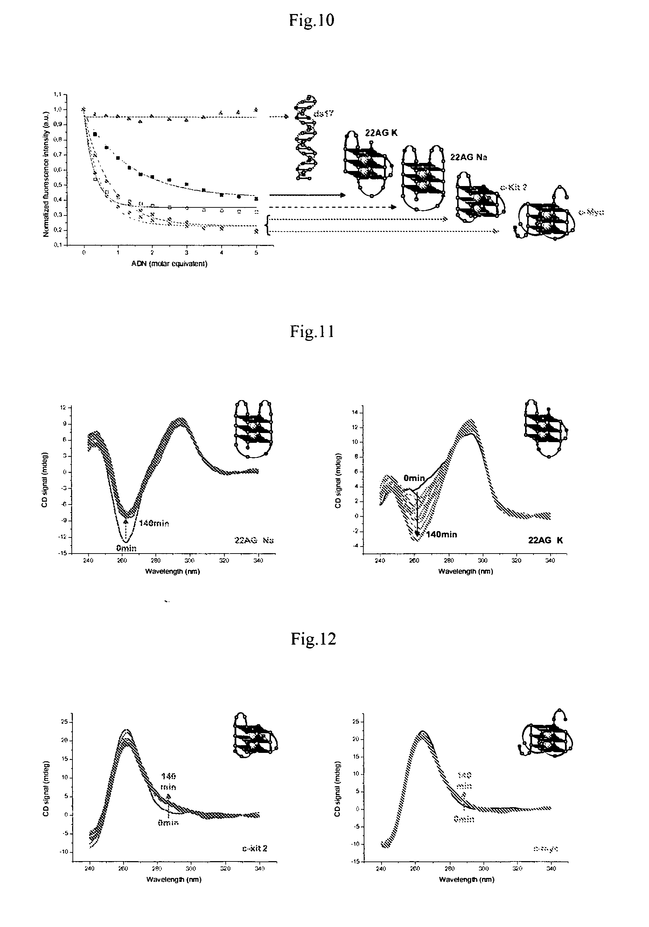

The various fluorescence titrations (λₑₓ = 340nm) are summarized in Fig. 10 which gives the graphical representation:

These data indicated that compound 3:
- dicerns very efficiently quadruplex- (22AG K, 22AG Na, c-kit 2 and c-myc) from duplex-DNA matrices (ds17), thus confirming FRET-melting results.
- interacts more efficiently and rapidly with 22AG Na, c-kit 2 and c-myc, while its interaction with 22AG K is weaker; this results are consistent with what has been observed *via* FRET-melting experiments, and represent a promising entry in the intra-quadruplex selectivity.

### Study II-4: Circular dichroism studies

Circular dichroism (CD) enables a deep study of the modification of the DNA structure upon the binding of a ligand; it thus reflects the affinity of the ligand to its target, and can also provide insight into its binding mode (Paramasivan et al, Methods, 2007, 43, 324).

### a) Quadruplex-interaction:

The quadruplex-DNA used herein is 22AG (see above), annealed both in a 10mM sodium cacodylate buffer pH 7.2, 100 mM NaCl (for 22AG Na) or KCl (for 22AG K). To a 3µM solution of 22AG in both buffers is added an excess of compound 3 (30µM, 10 equiv.). Th results are given in Fig.11. The interaction of compound 3 with quadruplexes is monitored as a function of time: it results in a modification of the amplitude of the CD signal of 22AG Na (left, especially at 263nm), while it reorganises completely the structure of 22AG K (right, especially at 263nm).

### b) Intra-quadruplex selectivity:

The intra-quadruplex selectivity is evaluated through CD by comparison of experiments carried out with 22AG (see above) and with two other quadruplex-structures: c-myc and c-kit2 (see above). The results are given on Fig. 11A and Fig.11B. To a 3µM solution of both c-kit2 (left) and c-myc (right) in cacodylate buffer (10mM sodium cacodylate + 100mM KCl) is added an excess of compound 3 (30µM, 10 equiv.). The interaction of compound 3 with quadruplexes is monitored as a function of time: it results in a modification of the amplitude of the CD signal of c-kit2 (especially at 285nm) and, to a lesser extent, of c-myc (285nm).

### c) Quadruplex-structure induction:

The equilibrium between folded and unfolded forms of 22AG oligonucleotide is highly sensitive to the presence and the nature of the buffer it is used in.Corresponding results are given in Fig.12A and Fig.12B. In low cationic buffer like 10mM Tris.HCl, pH 7.2, 22AG is mostly unfolded (random coiled); it is thus possible to influence the folded <-> unfolded equilibrium by adding compound 3. Thus, to a 3µM solution of random coiled 22AG in Tris.HCl buffer are added increasing amounts of compound 3 (from 0 to 7.5equiv.); it results in a deep modification of the CD signal of 22AG, with a disappearance of the random coiled-typical 257nm signal to the benefit of two novel maxima, one positive (near 290nm) and one negative (near 265nm), both being typical of the CD signature of 'anti-parallel' quadruplex-DNA structure (as 22AG Na, see above).

### d) Summary:

These data indicated that compound 3:
- interacts efficiently and almost spontaneously with 22AG Na, while in reacts efficiently but more slowly with 22AG K, which is consistent with fluorescence titrations and FRET-melting experiments results.
- interacts with c-myc and c-kit2, but its interaction does not modify the structure of these two quadruplexes.
- is able to shift the equilibrium between the unfolded and the folded form of 22AG toward the folded one.

### III- Biological methods and data:

### Study III-1: Inhibition of POT1 binding to telomeric sequence in vitro by compound 3

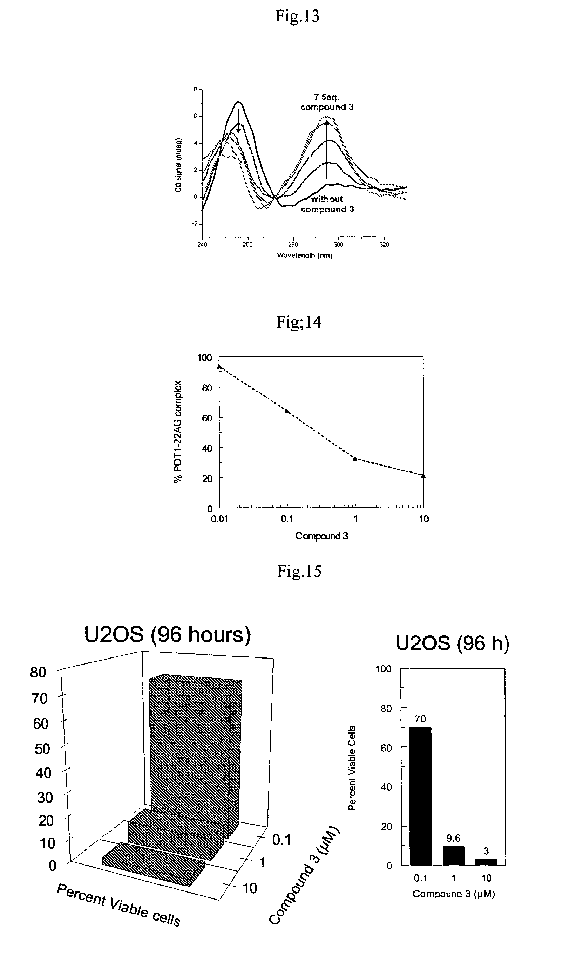

An electrophoretic mobility shift assay using hPOT1 was performed on the telomeric 22AG oligonucleotide (see above). 22AG was labeled at the 5' end with [γ-³²P]-ATP using T4 polynucletide kinase. Purified recombinant hPOT1 was produced in a baculovirus expression system. The POT1/22AG binding assay was performed in a total volume of 10µl containing 50 mM HEPES, pH 7.9, 100 mM NaCl, 0.1 mM EDTA, 4% w/v sucrose, 2% v/v glycerol, 0.1 mg/ml BSA, 0.02% w/v bromophenol blue, 30 nM hPOT1, 20 nM [α-³²P]-22AG. Different concentrations of compound 3 (10, 1, 0.1 and 0.01 µM) were added with hPOT1 to the solution and the mixture was incubated at room temperature for 30 min. Each individual sample was separated by electroporesis on 1% agarose gel in 0.5X Tris-Borate-EDTA buffer. The gel was run at 80V for35 min, dried on whatman DE81 paper and radiaoactivity visualized by a phosphorimager (Typhoon 9210, Amersham). Analysis of the data was carried out by ImageQuant software (Amersham) and results were expressed as a percentage of the POT1-22AG complex obtained in the untreated control (defined as 100%) (see Fig. 14).

These data indicated that compound 3 inhibits the binding of hPOT1 to telomeric 22AG sequence in a dose-dependent manner. In the experiment showed below the IC₅₀-_{POTI} for compound 3 is equal to 300 nM.

### Study III-2: Inhibition of cell proliferation by compound 3

The HT1080 human fibrosarcoma cell line stably transfected with pEGFP-POT1 vector (HT1080GFP-POT1) has been previously described (32) and U20S human osteosarcoma was from American Type Culture Collection (Rockville, USA). Cells were grown in Dublecco's modified Eagles's medium (Invitrogen) supplemented with 10% v/v fetal bovine serum and with 400µg/ml geneticin for HT1080GFP-POT1. Cells were plated in 6-wells culture plates on day 0 at 4.5 x10⁴ cells/well in the presence of different concentrations of compound 3 (10, 3, 1, 0.3 and 0.1 µM), each concentration in duplicate, and cultured for further 72 hours. At day 3, cells were washed with 1X PBS and trypsinized. For each treated cell sample, the number of viable cells was determined in the presence of trypan blue. Results are given on Fig. 15A and Fig.15B. They correspond to the relative cell number in percent as compared to control untreated cells defined as 100%.

These data indicates that compound 3 efficiently hampers the proliferation of cancer cells with an IC₅₀ comprised between 0.1 and 1µM.

Although the invention has been described above with reference to the disclosed embodiments, those skilled in the art will readily appreciate that the specific experiments detailed are only illustrative of the invention. Accordingly, the invention is limited only by the following claims.

### Bibliographic references

1 - Qin et al, 2008, Biochimie, 90, 1149
2- Hurley, Nature Reviews Cancer, 2002, 2, 188
3 - Neidle, et al, Nature Reviews Drug Discovery, 2002, 1, 383
4- Mergny et al, Nucleic Acids Res., 2002, 30, 839
5 - Rezler et al, Annu. Rev. Pharmacol. Toxicol., 2003, 43, 359
6 - Neidle et al, Nature Reviews Cancer, 2005, 5, 285;
7- Oganesian et al, BioEssay, 2007, 29, 155;
8 - De Cian et al, Biochimie, 2008, 90, 131; Monchaud et al, Org. Biomol. Chem., 2008, 6, 627; 9 9 - Ou et al, ChemMedChem, 2008, 3, 690)
10 - De Cian et al, Proc. Natl. Acad. Sci. U.S.A., 2007, 104, 17347
11(Tauchi et al, Oncogene, 2003, 22, 5338
12 - Tahara et al, Oncogene, 2006, 25, 1955)
13 - (Gomez et al, J. Biol. Chem., 2004, 279, 41487)
14 - (Gomez et al, J. Biol. Chem., 2006, 281, 38721)
15 - Gomez et al, Cancer Res., 2006, 66, 6908)
16 - (Doi et al, Org. Lett., 2006, 8, 4165)
17 - Tera et al, Heterocycles, 2006, 69, 505
18 - Barbieri et al, Nucleic Acids Res., 2007, 35, 3272
19 - Tera et al, Angew. Chem. Int. Ed., 2008, 47, 5557
20 - De Cian et al, Methods, 2007, 42, 183
21 - (De Cian et al, J. Am. Chem. Soc., 2007, 129, 1856)
22 - Qin et al, 2008, Biochimie, 90, 1149;
23 - Simonsson et al, Nucleic Acids Res., 1998, 26, 1167
24 - Ambrus et al, Biochemistry, 2005, 44, 2048
25 - Rangan et al, J. Biol. Chem., 2001, 276, 4640
26 - Siddiqui-Jain et al, Proc. Natl. Acad. Sci. U.S.A., 2002, 99, 11593) 27-(26).
28 - Teulade-Fichou et al, J. Am. Chem. Soc., 2003, 125, 4732
29 - Rankin et al, J. Am. Chem. Soc., 2005, 127, 10584;
30 - Fernando et al, Biochemistry, 2006, 45, 7854
31 - Paramasivan et al, Methods, 2007, 43, 324
32 - Gomez et al , J. Biol. Chem., 2006, 281, 38721

## Claims

1. Penta- hexa-, hepta-, octa-, nona- and, deca-heteroaryl derivatives, optionally isotopically-labelled, comprising
a combination of heterocycle 1 (Het-1)ₐ and/or heterocycle 2 (Het-2)_{b} and/ or heterocycle 3 (Het-3)_{c} and/or heterocycle 4 (Het-4)_{d} of formulae I, II, III and IV respectively, 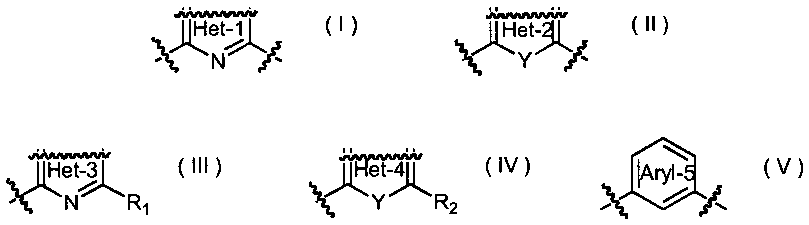 the N-oxides, the pharmaceutically acceptable addition salts,
- said combination comprising at least two different heterocyclic moeties, and optionally comprising an (Aryl-5)ₑ moiety of formula (V), and wherein one or several of said moieties are optionally substituted with one, two or three substituents, each independently selected from C₁₋₆alkyl ; C₁₋₄alkyloxy ; halo; hydroxy ; nitro; amino ; mono-or di(C₁₋₄alkyl)amino ; C₁₋₄alkylmethylamino ; morpholin-4-ylC₂₋₄alkyloxy; piperazin-1-ylC₂₋₄alkyloxy ; 4-C₁₋₄alkylpiperazin-1-yl C₂₋₄alkyloxy ; monocyclic or bicyclic ring selected from phenyl, benzyl, and naphthyl,
- a, b and e, being integers from 0 to 6, c and d are integers from 0 to 2, the sum a + b + c + d + e being ≤ 10
- Y is O or S;
Het-1 is a class of nitrogen heterocyclic-diyl ring selected in the group comprising 2,6-pyridin-diyl or 2,4-pyrimidin-diyl or 3,5-pyrazin-diyl or 2,4-(1,3,5-triazin)diyl or 3,5-(1,2,4-triazin)diyl or 2,4-oxazolin-diyl or 2,4-thiazolin-diyl,;
- Het-2 is a class of five-membered heterocyclic-diyl ring selected in the group comprising 2,5-oxazolin-diyl or 2,5-thiazolin-diyl, or 2,5-thiophen-diyl or 2,5-furan-diyl,;
- Het-3 and/ or Het-4 constitutes the endings of the said penta- to deca-heteroaryl derivatives,
wherein
- Het-3 is a class of nitrogen heterocyclic-yl ring selected from 2-pyridyl or 2-pyrimidyl or 4-pyrimidyl or 2-pyrazyl or 2-(1,3,5)triazyl or 3-(1,2,4)triazyl or 5-(1,2,4)triazyl or 4-oxazolyl or 4-thiazolyl ;
- Het-4 is a class of five-membered heterocyclic-yl selected from 2-oxazolyl or 5-oxazolyl or 2-thiazolyl or 5-thiazolyl or 2-thienyl or 2-furanyl 1;
- R₁ and R₂, are each independently selected from hydrogen; C₁₋₆alkyl; C₁₋₄alkyloxy ; halo; hydroxy; hydroxymethyl; nitro ; amino ; mono-or di(C₁₋₄alkyl)amino ; C₁₋₄alkylmethylamino ; mono-or di(C₁₋₄alkyl)amino(C₂₋₄alkyl)amino methyl; morpholin-4-yl C₂₋₄alkyloxy; piperazin-1-ylC₂₋₄alkyloxy; 4-C₁₋₄alkylpiperazin-1-yl, monocyclic or bicyclic selected from phenyl, benzyl, naphthyl.

2. The derivatives of claim 1, wherein one or several of said moieties are substituted with one, two or three substituents,each independently selected from C₁₋₆alkyl; C₁₋₄alkyloxy ; halo; hydroxy ; nitro; amino ; mono-or di(C₁₋₄alkyl)amino ; C₁₋₄alkylmethylamino ; morpholin-4-ylC₂₋₄alkyloxy ; piperazin-1-ylC₂₋₄alkyloxy ; 4-C₁₋₄alkylpiperazin-1-yl C₂₋₄alkyloxy ; monocyclic or bicyclic ring selected from phenyl, benzyl, naphthyl.

3. The derivatives of claim 1 or 2, comprising at least 5, at least 7, at least 8, at least 9 or 10 heterocyclic moieties.

4. The derivatives of claim 3, further comprising one or several 1,3-phenylene-diyl moieties.

5. The derivatives of anyone of claims 1 to 4, having one of the following structures:
- (Het-4 substituted by R2) - (Het-1) - (Het-4 substituted by R2)
- (Het-3 substituted by R1) -(Het-2)- (Het-3substituted by R1)
- (Het-3 substituted by R1) -(Het-2) -(Het-1) -(Het-2)- (Het-3 substituted by R1)
- (Het-3 substituted by R1) -(Het-2)-1,3-phenylene-diyl- (Het-2)-( (Het-3 substituted by R1)
- (1,3-phenylene-diyl- substituted by R1 or R2) -(Het-2) --(Het-1) --(Het-2) -(1,3-phenylene-diyl- substituted by R1 or R2)
- (Het-3 substituted by R1) -(Het-2)-(Het-1) -(Het-2)-( (Het-3 substituted by R1)
- (Het-3 substituted by R1) -(Het-2)-(Het-1)- (Het-1) -(Het-2)-( (Het-3 substituted by R1)

6. The derivatives of anyone of claims 1 to 5, wherein
. R1 and R2 are oxazolin- or pyridin-diyl groups, and/or
. Het-2 is an oxazolin-diyl group and/or
. Het-1 is a 2,6 pyridin-diyl, 2,6 pyrimidin-diyl, 2,6 pyrazin-diyl, or 1,3-phenylene-diyl-group.

7. A derivative according to anyone of claims 1 to 6, wherein one or more atoms are replaced by atoms having the same atomic number, but an atomic mass or mass number different from the atomic mass or mass number usually found in nature.

8. The derivatives according to anyone of claims 1 to 7 for use as drugs.

9. Pharmaceutical compositions comprising an effective amount of at least one derivative according to anyone of claims 1 to 7, in combination with a pharmaceutically acceptable carrier.

10. The pharmaceutical compositions of claim 9, under forms suitable for an administration by the oral or parenteral route.

11. The pharmaceutical compositions of claim 9 or 10, for use for treating cancer and infectious diseases, such as malaria.

12. The use of the derivatives according to anyone of claims 1-7, for making a drug for treating cancer or infectious diseases.

13. A method for preparing polyheteroaryl derivatives of anyone of claims 1 to 7, comprising
• reacting X1-A-X2
• with B-X3 or with X4-B-X3
wherein
- A is di- or tri- or tetra- heteroaryl-1,4 chain and B is a (mono- or di- or tri- or tetra-heteroaryl-1,4)-, the heteroaryl moieties optionally comprising one, two, three or four Aryl-5 moities or Aryl-5 terminal moieties
- Heteroaryl-1,4 being a combination of Het-1 and/or Het-2 and/or Het-3 and/or Het-4 which are as above defined,
- X1 and X2, identical or different, represent an hydrogen or halogen or triflate group,
- X3 represents an halogen or zinc halogenide or trialkyltin or boronate group
- X4 represents a carboxaldehyde group,
under conditions giving the desired polyheteroaryl derivatives.

14. Use of the derivatives of anyone of claims 1 to 7, as DNA quadruplex-specific probes.

15. Use of the derivatives of anyone of claims 1 to 7 to detect and/or purify quadruplex DNA or related nucleic acid structure.

## Patentansprüche

1. Penta- Hexa-, Hepta-, Octa-, Nona- und, Deca-Heteroarylderivate, ggf. isotopisch markiert, umfassend
- eine Kombination von Heterozyklus 1 (Het-1)ₐ und/oder Heterozyklus 2 (Het-2)_{b} und/ oder Heterozyklus 3 (Het-3)_{c} und/oder Heterozyklus 4 (Het-4)_{d} der Formeln I, II, III bzw. IV, 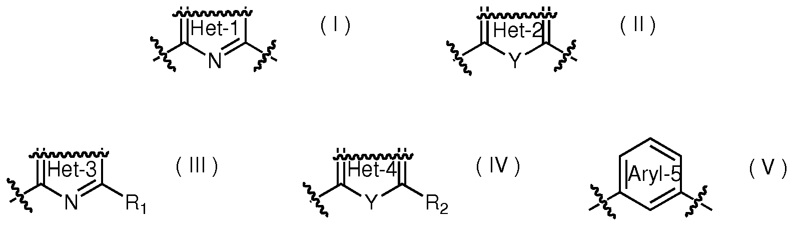 der N-oxide, der pharmazeutisch verträglichen Additionssalze,
- wobei diese Kombination mindestens zwei verschiedene heterozyklische Reste umfasst, und ggf. einen (Aryl-5)ₑ-Rest der Formel (V) umfasst, und wobei ggf. einer oder mehrere dieser Reste mit einem, zwei oder drei Substituenten substituiert sind, ein jeder unabhängig ausgewählt aus C₁₋₆Alkyl; C₁₋₄Alkyloxy; Halogen; Hydroxy ; Nitro; Amino ; Mono-oder Di(C₁₋₄alkyl)amino ; C₁₋₄Alkylmethylamino ; Morpholin-4-ylC₂₋₄Alkyloxy ; Piperazin-1-ylC₂₋₄alkyloxy ; 4-C₁₋₄Alkylpiperazin-1-yl C₂₋₄Alkyloxy ; monozyklischer oder bizyklischer Ring ausgewählt aus Phenyl, Benzyl, und Naphthyl,
- wobei a, b und e ganze Zahlen von 0 bis 6 sind, c und d ganze Zahlen von 0 bis 2 sind, wobei die Summe a+b+c+d+e ≤ 10 ist,
- Y O oder S ist;
Het-1 eine Klasse von Stickstoff-Heterozyklus-diyl-Ringen ist, die ausgewählt sind aus der Gruppe umfassend 2,6-Pyridin-diyl oder 2,4-Pyrimidin-diyl oder 3,5-Pyrazin-diyl oder 2,4-(1,3,5-Triazin)diyl oder 3,5-(1,2,4-Triazin)diyl oder 2,4-Oxazolin-diyl oder 2,4-Thiazolin-diyl,;
- Het-2 eine Klasse fünfgliedriger Heterozyklusdiyl-Ringen ist, die ausgewählt sind aus der Gruppe umfassend 2,5-Oxazolin-diyl oder 2,5-Thiazolin-diyl, oder 2,5-Thiophen-diyl oder 2,5-Furan-diyl,;
- Het-3 und/ oder Het-4 die Enden dieser Penta- bis Deca-Heteroarylderivate darstellen, wobei
- Het-3 eine Klasse von Stickstoff-Heterozyklus-yl-Ringen ist, die ausgewählt sind aus 2-Pyridyl oder 2-Pyrimidyl oder 4-Pyrimidyl oder 2-Pyrazyl oder 2-(1,3,5)Triazyl oder 3-(1,2,4)Triazyl oder 5-(1,2,4)Triazyl oder 4-Oxazolyl oder 4-Thiazolyl ;
- Het-4 eine Klasse fünfgliedriger Heterozyklus-yle ist, die ausgewählt sind aus 2-Oxazolyl oder 5-Oxazolyl oder 2-Thiazolyl oder 5-Thiazolyl oder 2-Thienyl oder 2-Furanyl 1 ;
- R₁ und R₂ unabhängig ausgewählt werden aus Wasserstoff; C₁₋₆Alkyl ; C₁₋₄Alkyloxy; Halogen; Hydroxy; Hhydroxymethyl; Nitro ; Amino ; Mono-oder Di(C₁₋₄alkyl)amino ; C₁₋₄Alkylmethylamino ; Mono-oder Di(C₁₋₄alkyl)amino(C₂₋₄alkyl)aminomethyl; Morpholin-4-yl C₂₋₄alkyloxy; Piperazin-1-ylC₂₋₄alkyloxy; 4-C₁-₄Alkylpiperazin-1-yl, monozyklischer oder bizyklischer ausgewählt aus Phenyl, Benzyl, Naphthyl.

2. Die Derivate nach Anspruch 1, wobei einer oder mehrere dieser Reste mit einem, zwei oder drei Substituenten substituiert sind, jeder unabhängig ausgesucht aus C₁₋₆Alkyl ; C₁₋₄Alkyloxy ; Halogen; Hydroxy ; Nitro; Amino ; Mono-oder Di(C₁₋₄alkyl)amino ; C₁₋₄Alkylmethylamino ; Morpholin-4-ylC₂₋₄alkyloxy ; Piperazin-1-ylC₂₋₄alkyloxy; 4-C₁₋₄alkylpiperazin-1-yl C₂₋₄alkyloxy ; monozyklischer oder bizyklischer Ring ausgewählt aus Phenyl, Benzyl, Naphthyl.

3. Die Derivate nach Anspruch 1 oder 2, umfassend mindestens 5, mindestens 7, mindestens 8, mindestens 9 oder mindestens 10 heterozyklische Reste.

4. Die Derivate nach Anspruch 3, ferner umfassend einen oder mehrere 1,3-Phenylen-diyl-Reste.

5. Die Derivate eines der Ansprüche 1 bis 4 mit einer der folgenden Strukturen:
- (Het-4 substituiert mit R2) - (Het-1) - (Het-4 substituiert mit R2)
- (Het-3 substituiert mit R1) -(Het-2)- (Het-3 substituiert mit R1)
- (Het-3 substituiert mit R1) -(Het-2) -(Het-1) -(Het-2)- (Het-3 substituiert mit R1)
- (Het-3 substituiert mit R1) -(Het-2)-1,3-Phenylen-diyl- (Het-2)-( (Het-3 substituiert mit R1)
- (1,3-Phenylen-diyl- substituiert mit R1 oder R2) -(Het-2) --(Het-1) --(Het-2) -(1,3-Phenylen-diyl- substituiert mit R1 oder R2)
- (Het-3 substituiert mit R1) -(Het-2)-(Het-1) -(Het-2)-( (Het-3 substituiert mit R1)
- (Het-3 substituiert mit R1) -(Het-2)-(Het-1)- (Het-1) -(Het-2)-( (Het-3 substituiert mit R1)

6. Die Derivate eines der Ansprüche 1 bis 5, wobei
. R1 und R2 Oxazolin- oder Pyridin-diyl-Gruppen sind, und/oder
. Het-2 eine Oxazolin-diyl-Gruppe ist und/oder
. Het-1 eine 2,6 Pyridin-diyl, 2,6 Pyrimidin-diyl, 2,6 Pyrazin-diyl, oder 1,3-Phenylen-diyl-Gruppe ist.

7. Ein Derivat gemäß einem der Ansprüche 1 bis 6, wobei ein oder mehrere Atome durch Atome mit derselben Atomzahl, aber mit einer von der üblicherweise in der Natur aufgefundenen Atommasse oder Massezahl abweichenden Atommasse oder Massezahl ersetzt sind.

8. Die Derivate gemäß einem der Ansprüche 1 bis 7 zur Verwendung als Arzneimittel.

9. Pharmazeutische Zusammensetzungen umfassend eine wirksame Menge mindestens eines Derivats gemäß einem der Ansprüche 1 bis 7, in Kombination mit einem pharmazeutisch verträglichen Träger.

10. Die pharmazeutischen Zusammensetzungen nach Anspruch 9, in Formen die geeignet sind für eine Verabreichung über den oralen oder parenteralen Weg.

11. Die pharmazeutischen Zusammensetzungen nach Anspruch 9 oder 10 zur Verwendung bei der Behandlung von Krebs und Infektionskrankheiten wie Malaria.

12. Die Verwendung der Derivate gemäß einem der Ansprüche 1-7 zur Herstellung eines Arzneimittels zur Behandlung von Krebs oder Infektionskrankheiten.

13. Ein Verfahren zur Herstellung von Polyheteroarylderivaten gemäß einem der Ansprüche 1 bis 7, umfassend:
• Umsetzen von X1-A- X2
• mit B-X3 oder mit X4-B-X3
wobei
- A eine Di- oder Tri- oder Tetra- Heteroaryl-1,4 Kette ist und B ein (Mono- oder Di- oder Tri- oder Tetra- Heteroaryl-1,4)- ist, wobei die Heteroaryl-Reste ggf. ein, zwei, drei oder vier Aryl-5-Reste oder Aryl-5 endständige Reste umfassen
- Heteroaryl-1,4 eine Kombination von Het-1 und/oder Het-2 und/oder Het-3 und/oder Het-4 ist, die wie oben definiert sind,
- X1 und X2, identisch oder unterschiedlich, eine Wasserstoff- oder eine Halogen- oder eine Triflatgruppe darstellen,
- X3 eine Halogen- oder eine Zinkhalogenid- oder eine Trialkyltin- oder Boronatgruppe darstellt
- X4 eine Carboxaldehyd-Gruppe darstellt,
unter Bedingungen, die die gewünschten Polyheteroarylderivate ergeben.

14. Verwendung der Derivate nach einem der Ansprüche 1 bis 7 als DNA-Quadruplex spezifische Sonden.

15. Verwendung der Derivate nach einem der Ansprüche 1 bis 7, um Quadruplex-DNA oder verwandte Nukleinsäurestrukturen nachzuweisen und/oder zu reinigen.

## Revendications

1. Dérivés penta-, hexa-, hepta-, octa-, nona- et déca-hétéroaryles, facultativement marqués isotopiquement, comprenant
- une combinaison d'un hétérocycle 1 (Het-1)ₐ et/ou d'un hétérocycle 2 (Het-2)_{b} et/ou d'un hétérocycle 3 (Het-3)_{c} et/ou d'un hétérocycle 4 (Het-4)_{d} de formules respectives I, II, III et IV, respectivement, 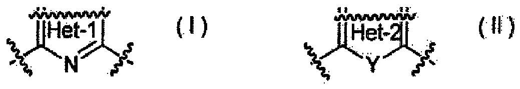 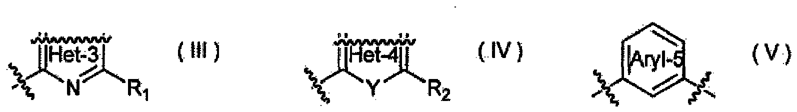 les N-oxydes, les sels d'addition pharmaceutiquement acceptables,
- ladite combinaison comprenant au moins deux fractions hétérocycliques différentes, et comprenant facultativement une fraction (Aryle-5)e de formule (V), et dans laquelle une ou plusieurs desdites fractions sont facultativement substituées avec un, deux ou trois substituants, chacun étant indépendamment sélectionné parmi un C₁₋₆alkyle; un C₁₋₄alkyloxy; un halo, un hydroxy, un nitro, un amino, un mono-ou di(C₁₋₄alkyl)amino ; un C₁₋₄alkylméthylamino; un morpholin-4-ylC₂₋₄alkyloxy ; un pipérazin-1-ylC₂₋₄alkyloxy ; un 4-C₁₋₄alkylpipérazin-1-yl C₂₋₄alkyloxy ; un cycle monocyclique ou bicyclique sélectionné parmi un phényle, un benzyle, et un naphtyle,
- a, b et e étant des nombres entiers de 0 à 6, c et d des nombres entiers de 0 à 2, la somme a + b + c + d + e étant ≤10
- Y est O ou S;
- Het-1 est une classe de cycles hétérocycliques azotés sélectionnés dans le groupe comprenant 2-6-pyridin-diyle ou 2,4-pyrimidin-diyle ou 3,5-pyrazin-diyle ou 2,4-(1,3,5-triazin)diyle ou 3,5-(1,2,4-triazin)diyle ou 2,4-oxazolin-diyle ou 2,4-thiazolin-diyle;
- Het-2 est une classe de cycles hétérocycliques à cinq chaînons sélectionnés dans le groupe comprenant 2,5-oxazolin-diyle, ou 2,5-thiazolin-diyle, ou 2,5-thiophen-diyle, ou 2,5-furan-diyle;
- Het-3 et/ou Het-4 constituent les extrémités desdits dérivés penta- à deca-hétéroaryles, dans lesquels
- Het-3 est une classe de cycles hétérocycliques azotés sélectionnés parmi 2-pyridyle ou 2-pyrimidyle ou 4-pyrimidyle ou 2-pyrazyle ou 2-(1,3,5)triazyle ou 3-(1,2,4)triazyle ou 5-(1,2,4)triazyle ou 4-oxazolyle ou 4-thiazolyle;
- Het-4 est une classe hétérocyctique à cinq chaînons sélectionnés parmi 2-oxazolyle ou 5-oxazolyle ou 2-thiazolyle ou 5-thiazolyle ou 2-thienyle ou 2-furanyle;
- R₁ et R₂, sont chacun indépendamment sélectionnés parmi un hydrogène, un C₁₋₆alkyle; un C₁₋₄alkyloxy ; un halo ; un hydroxy ; un hydroxyméthyle ; un nitro; un amino ; un mono-ou di (C₁₋₄alkyl)amino ; un C₁₋₄alkylméthylamino ; un mono-ou di(C₁₋₄alkyl)amino (C₂₋₄alkyl)aminométhyle; un morpholin-4-yl C₂₋₄alkyloxy ; un pipérazin-1-ylC₂₋₄alkyloxy ; un 4-C₁₋₄alkylpipérazin-1-yle, un monocyclique ou bicyclique sélectionné parmi un phényle, un benzyle, un naphtyle.

2. Dérivés selon la revendication 1, **caractérisés en ce que** une ou plusieurs desdites fractions sont substituées avec une, deux ou trois substituants, chacun indépendamment sélectionné parmi un C₁₋₆alkyle ; un C₁₋₄-alkyloxy ; un halo ; un hydroxy ; un nïtro ; un amino; un mono-ou di(C₁₋₄alkyl)amino ; un C₁₋₄alkylméthylamino; un morpholin-4-ylC₂₋₄alkyloxy; un pipérazin-1-ylC₂₋₄alkyloxy ; un 4-C₁₋₄alkylpipérazin-1-yl C₂₋₄alkyloxy ; un cycle monocyclique ou bicyclique sélectionné parmi un phényle, un benzyle, naphtyle.

3. Dérivés selon la revendication 1 ou 2, comprenant au moins 5, au moins 7, au moins 8, au moins 9 ou 10 fractions hétérocycliques.

4. Dérivés selon la revendication 3, comprenant en outre une ou plusieurs 1,3-fractions phenylene-diyle.

5. Dérivés selon l'une quelconque des revendications 1 à 4, ayant une des structures suivantes :
- (Het-4 substitué par R2) - (Het-1) - (Het-4 substitué par R2)
- (Het-3 substitué par R1) -(Het-2)- (Het-3 substitué par R1)
- (Het-3 substitué par R1) -(Het-2) -(Het-1) -(Het-2)- (Het-3 substitué par R1)
- (Het-3 substitué par R1) -(Het-2)-1,3-phenylene-diyl- (Het-2)-( (Het-3 substitué par R1)
- (1,3-phenylene-diyl- substitué par R1 ou R2) -(Het-2) -(Het-1) -(Het-2) -(1,3-phenylene-diyl- substitué par R1 ou R2)
- (Het-3 substitué par R1) -(Het-2)-(Het-1)-(Het-2)-( (Het-3 substitué par R1)
- (Het-3 substitué par R1) -(Het-2)-(Het-1)- (Het-1) -(Het-2)-( (Het-3 substitué par R1).

6. Dérivés selon l'une quelconque des revendications 1 à 5, **caractérisés en ce que**
. R1 et R2 sont des groupes oxazolin- ou pyridin-diyle, et/ou
. Het-2 est un groupe oxazolin-diyle et/ou
. Het-1 est un groupe 2,6-pyridin-diyle, 2,6-pyrimidin-diyle, 2,6-pyrazin-diyle, ou 1,3-phenylene-diyle.

7. Dérivés selon l'une quelconque des revendications 1 à 6, **caractérisés en ce que** un ou plusieurs atomes sont remplacés par des atomes ayant le même numéro atomique, mais avec une masse atomique ou un numéro de masse différent de la masse atomique ou du numéro de masse habituellement trouvé dans la nature.

8. Dérivés selon l'une quelconque des revendications 1 à 7 pour leur utilisation en tant que médicament.

9. Compositions pharmaceutiques comprenant une quantité efficace d'au moins un dérivé selon l'une quelconque des revendications 1 à 7, en combinaison avec un véhicule pharmaceutiquement acceptable.

10. Compositions pharmaceutiques selon la revendication 9, sous forme adaptées pour une administration par voie orale ou parentérale.

11. Compositions pharmaceutiques selon la revendication 9 ou 10, pour leur utilisation dans le traitement du cancer et des maladies infectieuses, telle que la malaria.

12. Utilisation des dérivés selon l'une quelconque des revendications 1-7, pour la préparation d'un médicament destiné au traitement du cancer ou des maladies infectieuses.

13. Procédé de préparation de dérivés polyhétéroaryles selon l'une quelconque des revendications 1 à 7, comprenant
. la réaction de X1-A-X2
. avec B-X3 ou X4-B-X3
**caractérisé en ce que**
- A est une di- ou tri- ou tétra- hétéroaryl-1,4 chaine et B est un (mono- ou di- ou tri- ou tétra-hétéroaryl-1,4)-, la fraction hétéroaryle comprenant facultativement un, deux, trois ou quatre fractions Aryl-5 ou fractions Aryl-5 terminales,
- Hétéroaryl-1,4 étant une combinaison de Het-1 et/ou Het-2 et/ou Het-3 et/ou Het-4 lesquels sont défini ci-dessus,
- X1 et X2, identiques ou différents, représentent un groupe hydrogène ou halogène ou triflate,
- X3 représente un halogène ou un halogénure de zinc ou de trialkylétain ou un groupe boronate,
- X4 représente un groupe carboxaldehyde,
dans des conditions donnant les dérivés polyhétéroaryles désirés.

14. Utilisation des dérivés selon l'une quelconque des revendications 1 à 7, en tant que sondes spécifiques de quadruplexe d'ADN.

15. Utilisation des dérivés selon l'une quelconque des revendications 1 à 7 pour détecter et/ou purifier un quadruplexe d'ADN ou la structure d'acide nucléique correspondante.


### SEQUENCE LISTING

<110> Institut Curie Centre National de la Recherche Scientique (CNRS) Institut National de la Santé et de la Recherche Médicale (INSERM) Muséum National d'Histoire Naturelle (MNHN)
<120> POLY-HETEROARYL DERIVATIVES, AND BIOLOGICAL APPLICATIONS
<130> CP 62955-3339
<150> EP 08 168 104.1
   <151> 2008-10-31
<160> 12
<170> PatentIn version 3.3
<210> 1
   <211> 6
   <212> DNA
   <213> Artificial Sequences
<220>
   <223> Oligonucleotides
<400> 1
   ttaggg 6
<210> 2
   <211> 21
   <212> DNA
   <213> Artificial Sequences
<220>
   <223> Oligonucleotides
<400> 2
   gggttagggt tagggttagg g 21
<210> 3
   <211> 24
   <212> DNA
   <213> Artificial Sequences
<220>
   <223> oligonucleotides
<400> 3
   gggtttaggg tttagggttt aggg 24
<210> 4
   <211> 24
   <212> DNA
   <213> Artificial Sequences
<220>
   <223> Oligonucleotides
<400> 4
   gggttcaggg ttcagggttc aggg 24
<210> 5
   <211> 26